# EUROPEAN PATENT SPECIFICATION

(11) **EP 3 094 301 B1**
(45) Date of publication and mention of the grant of the patent: **18.04.2018**
(21) Application number: 14777614.0
(22) Date of filing: 30.09.2014
(51) Int. Cl.: A61K 8/25, A61K 8/365, A61K 8/37, A61Q 1/04, A61K 8/81, A61K 8/92

(54) **LIQUID COMPOSITION COMPRISING A NON-VOLATILE OIL, A C18-C24 HYDROXYLATED FATTY ACID, AN ADDITIONAL WAX, A VINYLPYRROLIDONE COPOLYMER AND A SILICA AEROGEL**
FLÜSSIGE ZUSAMMENSETZUNG MIT EINEM NICHTFLÜCHTIGEN ÖL, EINER C18-C24-HYDROXYLIERTEN FETTSÄURE, EINEM ZUSÄTZLICHEN WACHS, EINEM VINYLPYRROLIDON-COPOLYMER UND EINEM SILICIUMDIOXID-AEROGEL
COMPOSITION LIQUIDE COMPRENANT UNE HUILE NON VOLATILE, UN ACIDE GRAS HYDROXYLÉ EN C18-C24, UNE CIRE SUPPLÉMENTAIRE, UN COPOLYMÈRE DE VINYLPYRROLIDONE ET UN AÉROGEL DE SILICE

(30) Priority: 01.10.2013 FR 1359520
(43) Date of publication of application: 23.11.2016
(73) Proprietor: L'Oréal, 75008 Paris (FR)
(72) Inventor: BOUARFA, Bouchra, F-75013 Paris (FR); GENDROT-ARCHIMBAUD, Alice, F-92290 Chatenay Malabry (FR)
(74) Representative: Wattremez, Catherine
(86) International application number: PCT/EP2014/070978
(87) International publication number: WO 2015/049252

(56) References cited:
- WO-A1-2011/148327
- WO-A2-2004/009037
- FR-A1- 2 964 566
- US-A1- 2003 232 030
- US-A1- 2005 175 570
- US-A1- 2011 020 263
- US-A1- 2011 147 259
- "Silica Silylate Aerogel for Cosmetic Applications", IP.COM JOURNAL, IP.COM INC., WEST HENRIETTA, NY, US, 30 January 2006 (2006-01-30), XP013112635, ISSN: 1533-0001
- DOW CORNING: "Dow Corning VM-2270 Aerogel Fine particles", INTERNET CITATION, April 2009 (2009-04), pages 1-5, XP002650585, Retrieved from the Internet: URL:http://www2.dowcorning.com/DataFiles/0 90007c88020e235.pdf [retrieved on 2011-07-15]

## Description

The present invention relates to a liquid cosmetic composition intended more particularly for making up and/or caring for the lips, comprising at least one non-volatile oil, 12-hydroxystearic acid, an additional wax, at least one vinylpyrrolidone/olefin copolymer and at least one hydrophobic silica aerogel.

The development of fluid compositions dedicated to making up and/or caring for the skin and/or the lips, especially the lips, such as lip glosses (liquid lipstick), which are stable and endowed with satisfactory properties in terms of application (glidance on application, ease of spreading and fineness of the deposit) and also in terms of the makeup effect of the deposit on the lips, for instance the gloss and/or the gloss persistence, preferably without becoming tacky, is an ongoing objective.

In general, formulations corresponding to liquid presentation forms (also known as fluids), for example of "gloss" type in the case of lip compositions, conventionally comprise fillers, such as silica, and in particular nanosilicas, inter alia, to thicken the composition and to obtain a fluid and stable texture, which may be readily and uniformly applied to the skin or the lips.

Specifically, in the case of liquid compositions that especially have, in point of fact, the advantage of enabling the use of large amounts of oils, it is necessary to find a means for thickening these oils in order to obtain a texture that is stable over time and of intermediate viscosity, i.e. which is not too liquid (since it would then be difficult to apply and/or would risk running and/or migrating into the wrinkles and fine lines around the lips), and which is not too thick either, since it would then prove to be difficult to spread on the skin and/or the lips. It is also sought to obtain a composition whose deposition on the skin or the lips is fine and does not give rise to a greasy sensation (in the case of an excessively oily deposit) or a sensation of dryness or tautness (in the case of a dry deposit).

In the case of compositions intended for making up the lips, these liquid formulations, of "gloss" type, are preferred for affording an optimized gloss effect, generally by virtue of the presence of oils with a high refractive index. In the case of such compositions, it is necessary to find a means for thickening these oils without impairing this gloss effect.

In general, the starting materials, and in particular filler, conventionally used at the present time for sufficiently thickening a composition, in particular for holding the pigments and nacres in suspension, are "nanosilicas" (the term "nanosilicas" means particles of nanometric size or comprising at least a fraction of nanometric size), generally chosen from the fumed silica particles of INCI name Silica Dimethyl Silylate, which may be hydrophilic- or hydrophobic-treated, for example such as the compound sold under the reference Aerosil® R 972 by Evonik Degussa.

The use of nanosilicas also generally makes it possible to obtain optimized application properties such as destructuring under the effect of the shear generated by the application, which makes it possible to deposit the product uniformly onto the lips, followed by restructuring of the deposit after application, allowing satisfactory remanence of the cosmetic result, and/or making it possible to prevent or limit the unaesthetic migration of the product in the fine lines around the lips. Thus, standard makeup compositions, and in particular lip glosses, conventionally comprise between 2% and 7% by weight of nanosilicas (often hydrophobic-treated), in order to efficiently thicken the oils.

However, as soon as an attempt is made to dispense with the presence of "nanosilicas", the compositions that are obtained are liquid oily mixtures that are not sufficiently thickened and/or gelled. In particular, such compositions do not make it possible to maintain pigments and/or nacres in suspension in the composition. Sedimentation of these compounds to the bottom of the composition is then observed.

Moreover, the deposit of such "nanosilica"-free compositions onto the skin and/or the lips generally has the drawback of running and/or of migrating more or less substantially into the wrinkles and fine lines after application.

Furthermore, these compositions very often have a tacky and/or greasy nature, which is especially induced by the presence of insufficiently gelled oils; this tacky nature is especially reflected by the made-up lips adhering together, which is thus unpleasant in terms of comfort for the user.

An alternative means to the "nanosilicas" used hitherto is thus sought, to obtain a makeup and/or care composition, in particular a makeup composition, in which the oils are sufficiently gelled and/or thickened, so as not to have the drawbacks mentioned previously, in particular a composition which is stable and which has good spreading properties and whose deposit on the skin and/or the lips, in particular on the lips, is fine, homogeneous and glossy.

Moreover, compositions whose deposit on the skin and/or the lips does not have a tacky nature are also sought. Specifically, the deposits obtained with liquid formulations comprising a large content of oil, in particular in the case of liquid compositions such as lip glosses, very often have a tacky nature, which is especially induced by the use of these oils, this tacky nature being reflected especially by the made-up lips adhering together, which is thus unpleasant in terms of comfort for the user.

It has been found, surprisingly, that a combination of waxes chosen from linear C₁₈-C₂₄ hydroxylated fatty acids with other waxes, a PVP/α-olefin copolymer, in the presence of non-volatile oil(s) and of a hydrophobic silica aerogel, makes it possible to obtain stable liquid cosmetic compositions that have good application properties and whose deposit has satisfactory gloss, is comfortable (fine deposit, no greasy, pasty and/or dry feel), homogeneous and sparingly tacky or non-tacky.

Thus, according to one of its aspects, the present invention is directed towards a liquid cosmetic composition, preferably for making up and/or caring for the lips, comprising, in a physiologically acceptable medium, at least one fatty phase comprising:
- at least one non-volatile oil,
- at least one linear C₁₈-C₂₄ hydroxylated fatty acid, preferably such as 12-hydroxystearic acid,
- at least one additional wax other than the linear C₁₈-C₂₄ hydroxylated fatty acid,
- at least one copolymer of vinylpyrrolidone and of at least one C₂-C₄₀ α-olefin,
- at least hydrophobic silica aerogel particles,
- the said composition optionally comprising water at less than 5% by weight relative to the total weight of the composition, and preferably being anhydrous.

Surprisingly, it has been found that such a cosmetic composition for making up and/or caring for the lips or the skin is stable and makes it possible to obtain a deposit on the lips and/or the skin that is homogeneous, fine, non-tacky and glossy.

Moreover, the composition according to the invention is homogeneous and stable at room temperature. The term "stable" composition means especially that the composition does not show any phase separation or exudation in particular after 24 hours. Moreover, the term "stable" means especially that the composition according to the invention must not show any sedimentation of the particles present, for example of the pigments and/or nacres, when the composition comprises such compounds. In particular, no sedimentation of the pigments and/or nacres must be observed after 24 hours at 25°C.

Preferably, the term "stable" also means that no sedimentation of the pigments and/or nacres should be observed after the composition according to the invention has been subjected to centrifugation at 450×g for 10 minutes.

Moreover, the composition according to the invention is easy to apply to the skin and/or the lips. The ease of application is especially reflected in terms of the glidance and/or the ease of spreading and also by the ease of obtaining a fine, homogeneous deposit.

The composition according to the invention is in liquid form at room temperature. For the purposes of the present invention, the term "liquid" characterizes the state of a composition at room temperature (25°C) and at atmospheric pressure (760 mmHg). The term "liquid" especially means a fluid composition, as opposed to a solid composition.

In a particularly preferred manner, the composition according to the invention is a makeup composition, preferably for the lips, such as a lip gloss.

According to another aspect, the present invention relates to a cosmetic process for making up and/or caring for the lips, comprising the application to the lips and/or the skin of a cosmetic composition as defined previously. Particularly preferably, the invention relates to a process for making up, preferably the lips, comprising the application to the lips of a cosmetic composition as defined previously.

In the text hereinbelow, the expression "*at least one*" is equivalent to "one or more" and, unless otherwise indicated, the limits of a range of values are included in that range.

### PHYSIOLOGICALLY ACCEPTABLE MEDIUM

The term "physiologically acceptable medium" is intended to denote a medium that is particularly suitable for the application of a composition of the invention to the skin or the lips.

The physiologically acceptable medium is generally adapted to the nature of the support onto which the composition has to be applied, and also to the appearance under which the composition has to be packaged.

The composition according to the invention comprises less than 5% by weight of water relative to the total weight of the composition. Preferably, the composition according to the invention comprises less than 2% by weight of water relative to the total weight of the composition. Particularly preferably, the composition according to the invention is anhydrous. The term "anhydrous" especially means that water is preferably not deliberately added to the compositions, but may be present in trace amounts in the various compounds used in the compositions.

### FATTY PHASE

### LINEAR C₁₈-C₂₄ HYDROXYLATED FATTY ACID

The composition according to the invention comprises at least one linear C₁₈-C₂₄ hydroxylated fatty acid, which is preferably saturated. Preferably, the linear C₁₈-C₂₄ hydroxylated fatty acid is 12-hydroxystearic acid. This compound is sold especially under the reference 12-Hydroxystearic acid Premium Grade 12H-P by the company Thai Kawaken.

According to a preferred embodiment, the composition according to the invention comprises a total content of linear C₁₈-C₂₄ hydroxylated fatty acid(s) ranging from 0.1% to 5% by weight, better still preferably from 0.1% to 4% by weight and preferably from 0.5% to 3% by weight relative to the total weight of the composition. Preferably, 12-hydroxystearic acid is present in a content between 0.1% and 5% by weight, preferably from 0.1% to 4% by weight and preferably from 0.5% to 3% by weight relative to the total weight of the composition.

### ADDITIONAL WAXES

The composition according to the invention comprises at least one additional wax other than the linear C₁₈-C₂₄ hydroxylated fatty acid.

According to a preferred embodiment, the content of additional wax(es) is between 0.1% and 10% by weight, more particularly between 0.5% and 7% by weight and even more preferably between 0.5% and 5% by weight, relative to the total weight of the composition.

Advantageously, the total content of waxes is not more than 12% by weight, preferably not more than 10% by weight and even more advantageously not more than 7% by weight, relative to the total weight of the composition. More particularly, the minimum wax content is preferably at least 0.2% by weight, preferably at least 0.6% by weight and even more advantageously at least 1% by weight, relative to the weight of the composition.

The term "wax" under consideration in the context of the present invention generally means a lipophilic compound that is solid at room temperature (25°C), with a solid/liquid reversible change of state, having a melting point of greater than or equal to 30°C, which may be up to 200°C and in particular up to 120°C.

For the purposes of the invention, the melting point corresponds to the temperature of the most endothermic peak observed in thermal analysis (DSC) as described in the standard ISO 11357-3; 1999. The melting point of the wax may be measured using a differential scanning calorimeter (DSC), for example the calorimeter sold under the name MDSC 2920 by the company TA Instruments.

The measuring protocol is as follows:
A sample of 5 mg of wax placed in a crucible is subjected to a first temperature rise ranging from -20°C to 100°C, at a heating rate of 10°C/minute, it is then cooled from 100°C to -20°C at a cooling rate of 10°C/minute and is finally subjected to a second temperature increase ranging from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

Preferably, the additional wax(es) are chosen from waxes with a melting point of greater than or equal to 60°C and preferably greater than or equal to 65°C.

The additional waxes that may be used in the compositions according to the invention are chosen from waxes that are solid at room temperature of animal, plant, mineral or synthetic origin, and mixtures thereof.

The composition according to the invention may comprise at least one additional polar or apolar wax, or mixtures thereof.

### Polar waxes:

According to a first embodiment of the invention, the additional wax is a polar wax.

For the purposes of the present invention, the term "polar wax" means a wax whose solubility parameter at 25°C, δₐ, is other than 0 (J/cm³)^{½}. In addition, the said polar waxes have a reversible solid/liquid change of state, and also the melting point characteristics mentioned previously.

In particular, the term "polar wax" means a wax whose chemical structure is formed essentially from, or even constituted of, carbon and hydrogen atoms, and comprising at least one highly electronegative heteroatom such as an oxygen, nitrogen, silicon or phosphorus atom.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:
- δ_{D} characterizes the London dispersion forces derived from the formation of dipoles induced during molecular impacts;
- δₚ characterizes the Debye interaction forces between permanent dipoles and also the Keesom interaction forces between induced dipoles and permanent dipoles;
- δₕ characterizes the specific interaction forces (such as hydrogen bonding, acid/base, donor/acceptor, etc.); and
- δₐ is determined by the equation: δₐ=(δₚ² + δₕ²)^{½}

The parameters δₚ, δₕ, δ_{D} and δₐ are expressed in (J/cm³)^{½}.

The polar waxes may especially be hydrocarbon-based, fluoro or silicone waxes.

The term "silicone wax" means a wax comprising at least one silicon atom, especially comprising Si-O groups.

The term "hydrocarbon-based wax" is intended to mean a wax formed essentially from, or even constituted of, carbon and hydrogen atoms, and optionally oxygen and nitrogen atoms, and that does not contain any silicon or fluorine atoms. It may contain alcohol, ester, ether, carboxylic acid, amine and/or amide groups.

According to a first preferred embodiment, the polar wax is a hydrocarbon-based wax. As a hydrocarbon-based polar wax, a wax chosen from ester waxes and alcohol waxes is in particular preferred.

The expression "ester wax" is understood according to the invention to mean a wax comprising at least one ester function.

The expression "alcohol wax" is understood according to the invention to mean a wax comprising at least one alcohol function, i.e. comprising at least one free hydroxyl (OH) group.

According to a first embodiment, the polar wax is chosen from ester waxes, alcohol waxes and silicone waxes.

Preferably, the ester wax is chosen from:
i) waxes of formula R₁COOR₂ in which R₁ and R₂ represent linear, branched or cyclic aliphatic chains, the number of atoms of which varies from 10 to 50, which may contain a heteroatom such as O, N or P. In particular, use may be made, as an ester wax, of a C₂₀-C₄₀ alkyl (hydroxystearyloxy)stearate (the alkyl group comprising from 20 to 40 carbon atoms), alone or as a mixture, or a C₂₀-C₄₀ alkyl stearate. Such waxes are especially sold under the names Kester Wax K 82 P®, Hydroxypolyester K 82 P®, Kester Wax K 80 P® and Kester Wax K82H by the company Koster Keunen.
   Use may also be made of a glycol and butylene glycol montanate (octacosanoate) such as the wax Licowax KPS Flakes (INCI name: glycol montanate) sold by the company Clariant.
ii) Bis(1,1,1-trimethylolpropane) tetrastearate, sold under the name Hest 2T-4S® by the company Heterene,
iii) diester waxes of a dicarboxylic acid of general formula R³-(-OCO-R⁴-COO-R⁵), in which R³ and R⁵ are identical or different, preferably identical, and represent a C₄-C₃₀ alkyl group and R⁴ represents a linear or branched C₄-C₃₀ aliphatic group which may or may not comprise one or more unsaturations and which is preferably linear and unsaturated,
iv) the waxes corresponding to the partial or total esters, preferably the total esters, of a saturated, optionally hydroxylated C₁₆-C₃₀ carboxylic acid, with glycerol. The term "total esters" means that all the hydroxyl functions of glycerol are esterified.
   By way of example, mention may be made of trihydroxystearine (or glyceryl trihydroxystearate), tristearine (or glyceryl tristearate) and tribehenine (or glyceryl tribehenate), alone or as a mixture.
v) Mention may also be made of the waxes obtained by catalytic hydrogenation of animal or plant oils having linear or branched C₈-C₃₂ fatty chains, for example such as hydrogenated jojoba oil, hydrogenated sunflower oil, hydrogenated castor oil, hydrogenated coconut oil, and also the waxes obtained by hydrogenation of castor oil esterified with cetyl alcohol, such as those sold under the names Phytowax Ricin 16L64® and 22L73® by the company Sophim. Such waxes are described in patent application FR-A-2792190 and the waxes obtained by hydrogenation of olive oil esterified with stearyl alcohol such as that sold under the name Phytowax Olive 18L57, or else;
vi) beeswax, synthetic beeswax, polyglycerolated beeswax, carnauba wax, candelilla wax, oxypropylenated lanolin wax, rice bran wax, ouricury wax, esparto grass wax, cork fibre wax, sugar cane wax, Japan wax, sumach wax; montan wax, orange wax, laurel wax and hydrogenated jojoba wax.
vii) mixtures thereof.
   According to another embodiment, the polar wax may be an alcohol wax. More particularly, these waxes are preferably linear, preferably saturated C₁₆-C₅₀ and advantageously C₁₆-C₄₀ fatty alcohols and optionally comprise at least one free hydroxyl. The said waxes may also be polyoxyethylenated. Alcohol waxes that may be mentioned include for example the wax Performacol 550-L Alcohol from New Phase Technologies, stearyl alcohol and cetyl alcohol.

According to a second embodiment, the polar wax may be a silicone wax, for instance siliconized beeswax.

Preferably, the additional wax is a polar wax chosen from the waxes corresponding to the total esters of a saturated, optionally hydroxylated C₁₆-C₃₀ carboxylic acid with glycerol, such as trihydroxystearine; beeswax; synthetic beeswax; polyglycerolated beeswax; carnauba wax; candelilla wax; oxypropylenated lanolin wax; rice bran wax; ouricury wax; esparto grass wax; cork fibre wax; sugar cane wax; Japan wax; sumach wax; montan wax; orange wax; laurel wax; hydrogenated jojoba wax, alone or as a mixture.

### Apolar waxes:

According to another embodiment, the additional wax is an apolar wax.

For the purposes of the present invention, the term "apolar wax" means a wax whose solubility parameter at 25°C as defined below, δₐ, is equal to 0 (J/cm³)^{½}. In addition, the said apolar waxes have a reversible solid/liquid change of state, and also the melting point characteristics mentioned previously.

Apolar waxes are in particular hydrocarbon-based waxes constituted solely of carbon and hydrogen atoms, and free of heteroatoms such as N, O, Si and P.

In particular, the expression "apolar wax" is understood to mean a wax that is constituted solely of apolar wax, rather than a mixture also comprising other types of waxes that are not apolar waxes.

As illustrations of apolar waxes that are suitable for use in the invention, mention may be made especially of hydrocarbon-based waxes, for instance microcrystalline waxes, paraffin waxes, ozokerite, polymethylene waxes and polyethylene waxes.

Polyethylene waxes that may be mentioned include Performalene 500-L Polyethylene and Performalene 400 Polyethylene sold by New Phase Technologies.

A polymethylene wax that may be mentioned is Cirebelle 108 sold by Cirebelle.

An ozokerite that may be mentioned is Ozokerite Wax SP 1020 P.

As microcrystalline waxes that may be used, mention may be made of Multiwax W 445® sold by the company Sonneborn, and Microwax HW® and Base Wax 30540® sold by the company Paramelt.

As microwaxes that may be used in the compositions according to the invention as apolar wax, mention may be made especially of polyethylene microwaxes such as those sold under the names Micropoly 200®, 220®, 220L® and 250S® by the company Micro Powders.

According to a preferred embodiment, the composition according to the invention comprises at least one additional wax chosen from apolar waxes.

Preferably, the additional apolar wax(es) are chosen from polyethylene waxes, ozokerites, microcrystalline waxes and polymethylene waxes, alone or as mixtures.

### OILS

The composition according to the invention comprises at least one non-volatile oil.

The term "*oil*" means a water-immiscible non-aqueous compound that is liquid at room temperature (25°C) and at atmospheric pressure (760 mmHg).

In particular, the oil may be chosen from hydrocarbon-based oils, silicone oils and/or fluoro oils, and mixtures thereof.

Preferentially, the non-volatile oil may be chosen from hydrocarbon-based oils and/or silicone oils.

### NON-VOLATILE OILS

The term *"non-volatile"* oil refers to an oil for which the vapour pressure at room temperature and atmospheric pressure is non-zero and less than 0.02 mmHg (2.66 Pa) and better still less than 10⁻³ mmHg (0.13 Pa).

The non-volatile oils may be hydrocarbon-based oils especially of plant origin, oils of synthetic or mineral origin, silicone oils, fluoro oils, or mixtures thereof.

### Apolar oils:

According to a first embodiment, the said non-volatile oil may be an apolar oil, preferably an apolar hydrocarbon-based oil.

These oils may be of plant, mineral or synthetic origin.

For the purposes of the present invention, the term "apolar oil" is intended to mean an oil of which the solubility parameter at 25°C, δₐ, is equal to 0 (J/cm³)^{1/2}.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:
- δ_{D} characterizes the London dispersion forces derived from the formation of dipoles induced during molecular impacts;
- δₚ characterizes the Debye interaction forces between permanent dipoles and also the Keesom interaction forces between induced dipoles and permanent dipoles;
- δₕ characterizes the specific interaction forces (such as hydrogen bonding, acid/base, donor/acceptor, etc.); and
- δₐ is determined by the equation: δₐ= (Dₚ² + δₕ²)^{½}.

The parameters δₚ, δₕ, δ_{D} and δₐ are expressed in (J/cm³)^{½}.

The term "hydrocarbon-based oil" means an oil formed essentially from, or even constituted of, carbon and hydrogen atoms, and optionally oxygen and nitrogen atoms, and not containing any silicon or fluorine atoms. It may contain alcohol, ester, ether, carboxylic acid, amine and/or amide groups.

Preferably, the non-volatile apolar hydrocarbon-based oil may be chosen from linear or branched hydrocarbons of mineral or synthetic origin, such as:
- liquid paraffin or derivatives thereof,
- squalane,
- isoeicosane,
- naphthalene oil,
- polybutylenes such as Indopol H-100 (molar mass or MW = 965 g/mol), Indopol H-300 (MW = 1340 g/mol) and Indopol H-1500 (MW = 2160 g/mol) sold or manufactured by the company Amoco,
- polyisobutenes,
- hydrogenated polyisobutylenes such as Parleam® sold by the company Nippon Oil Fats, Panalane H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol), or alternatively Parleam Lite sold by NOF Corporation,
- decene/butene copolymers, polybutene/polyisobutene copolymers, in particular Indopol L-14,
- polydecenes and hydrogenated polydecenes such as: Puresyn 10 (MW = 723 g/mol) and Puresyn 150 (MW = 9200 g/mol) sold or manufactured by the company Mobil Chemicals, or alternatively Puresyn 6 sold by ExxonMobil Chemical),
- and mixtures thereof.

Preferably, the composition according to the invention comprises at least one apolar oil preferably chosen from polybutenes, polyisobutenes, hydrogenated polyisobutenes, polydecenes and/or hydrogenated polydecenes, and mixtures thereof.

According to a preferred embodiment, a composition in accordance with the invention comprises at least one apolar hydrocarbon-based oil preferably chosen from hydrogenated polyisobutylene and hydrogenated polydecene.

Preferably, the content of apolar volatile oil(s) in the composition, if it comprises any, ranges from 5% to 60% by weight, for example from 10% to 45% by weight, relative to the total weight of the composition.

### Polar oils:

According to another particular embodiment, the composition comprises at least one non-volatile polar oil. The said oil may be a hydrocarbon-based oil, silicone oil or fluoro oil.

Preferentially, the said non-volatile oil is a polar hydrocarbon-based.

The term "silicone oil" means an oil containing at least one silicon atom, and in particular containing Si-O groups.

The term "fluoro oil" means an oil containing at least one fluorine atom.

These oils may be of plant, mineral or synthetic origin.

The term "hydrocarbon-based oil" is intended to mean an oil formed essentially from, or even constituted by, carbon and hydrogen atoms, and possibly oxygen and nitrogen atoms, and not containing any silicon or fluorine atoms. It may contain alcohol, ester, ether, carboxylic acid, amine and/or amide groups.

For the purposes of the present invention, the term "*polar oil*" means an oil whose solubility parameter at 25°C, δₐ, is other than 0 (J/cm³)^{1/2}.

In particular, the hydrocarbon-based non-volatile polar oil may be chosen from the list of oils below, and mixtures thereof:
- hydrocarbon-based plant oils such as liquid triglycerides of fatty acids containing from 4 to 10 carbon atoms, for instance heptanoic or octanoic acid triglycerides or jojoba oil;
- ester oils, preferably chosen from:
- fatty acid esters, in particular of 4 to 22 carbon atoms, and especially of octanoic acid, heptanoic acid, lanolic acid, oleic acid, lauric acid or stearic acid, for instance propylene glycol dioctanoate, propylene glycol monoisostearate or neopentyl glycol diheptanoate;
- synthetic esters, for instance the oils of formula R₁COOR₂ in which R₁ represents a linear or branched fatty acid residue comprising from 4 to 40 carbon atoms and R₂ represents a hydrocarbon-based chain, which is especially branched, containing from 4 to 40 carbon atoms, on condition that R₁ + R₂ ≥ 16, for instance purcellin oil (cetostearyl octanoate), isononyl isononanoate, C₁₂ to C₁₅ alkyl benzoate, 2-ethylhexyl palmitate, octyldodecyl neopentanoate, 2-octyldodecyl stearate, 2-octyldodecyl erucate, oleyl erucate, isostearyl isostearate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate, 2-octyldodecyl myristate or 2-diethylhexyl succinate; preferably, the preferred synthetic esters R₁COOR₂ in which R₁ represents a linear or branched fatty acid residue comprising from 4 to 40 carbon atoms and R₂ represents a hydrocarbon-based chain, which is especially branched, containing from 4 to 40 carbon atoms are such that R₁ and R₂ ≥ 20;
- linear fatty acid esters with a total carbon number ranging from 35 to 70, for instance pentaerythrityl tetrapelargonate (MW = 697 g/mol);
- hydroxylated esters, preferably with a total carbon number ranging from 35 to 70, for instance polyglyceryl-2 triisostearate (MW = 965 g/mol), isostearyl lactate, octyl hydroxystearate, octyldodecyl hydroxystearate, diisostearyl malate, glyceryl stearate; diethylene glycol diisononanoate;
- esters of aromatic acids and of alcohols comprising 4 to 22 atoms, such as tridecyl trimellitate (MW = 757 g/mol);
- C₂₄-C₂₈ esters of branched fatty alcohols or fatty acids such as those described in patent application EP-A-0 955 039, and especially triisoarachidyl citrate (MW = 1033.76 g/mol), pentaerythrityl tetraisononanoate (MW = 697 g/mol), glyceryl triisostearate (MM = 891 g/mol), glyceryl tris(2-decyl)tetradecanoate (MW = 1143 g/mol), pentaerythrityl tetraisostearate (MW = 1202 g/mol), polyglyceryl-2 tetraisostearate (MW = 1232 g/mol) or pentaerythrityl tetrakis(2-decyl)tetradecanoate (MW = 1538 g/mol),
- polyesters resulting from the esterification of at least one hydroxylated carboxylic acid triglyceride with an aliphatic monocarboxylic acid and with an aliphatic dicarboxylic acid, which is optionally unsaturated, for instance the succinic acid and isostearic acid castor oil sold under the reference Zénigloss by Zénitech;
- esters of a diol dimer and of a diacid dimer of general formula HO-R¹-(-OCO-R²-COO-R¹-)ₕ-OH, in which:
   R¹ represents a diol dimer residue obtained by hydrogenation of dilinoleic diacid,
   R² represents a hydrogenated dilinoleic diacid residue, and
   h represents an integer ranging from 1 to 9,
   especially the esters of dilinoleic diacids and of dilinoleyl diol dimers sold by the company Nippon Fine Chemical under the trade names Lusplan DD-DA5® and DD-DA7®, and
- polyesters obtained by condensation of an unsaturated fatty acid dimer and/or trimer and of diol, such as those described in patent application FR 0 853 634, in particular such as dilinoleic acid and 1,4-butanediol. Mention may especially be made in this respect of the polymer sold by Biosynthis under the name Viscoplast 14436H (INCI name: dilinoleic acid/butanediol copolymer), or copolymers of polyols and of diacid dimers, and esters thereof, such as Hailuscent ISDA;
- fatty alcohols containing from 12 to 26 carbon atoms, which are preferably branched, for instance octyldodecanol, 2-butyloctanol, 2-hexyldecanol, 2-undecylpentadecanol and oleyl alcohol;
- C₁₂-C₂₆ fatty acids, such as oleic acid, linoleic acid and linolenic acid, and mixtures thereof;
- oils of plant origin such as sesame oil (820.6 g/mol); and the C18-36 acid triglyceride (Dub TGI 24 from Stéarineries Dubois);
- dialkyl carbonates, the two alkyl chains possibly being identical or different, such as dicaprylyl carbonate sold under the name Cetiol CC® by Cognis; and

Preferably, the polar non-volatile hydrocarbon-based oil is chosen from hydrocarbon-based oils from plants or of plant origin, ester oils, fatty alcohols containing from 12 to 26 carbon atoms and fatty acids containing from 12 to 26 carbon atoms, and mixtures thereof.

Preferably, the composition according to the invention comprises at least one non-volatile oil chosen from synthetic esters of formula R₁COOR₂ in which R₁ represents a linear or branched fatty acid residue comprising from 4 to 40 carbon atoms and R₂ represents a hydrocarbon-based chain that is especially branched, containing from 4 to 40 carbon atoms, provided that R₁ + R₂ ≥ 16.

Preferably, the composition according to the invention comprises at least one non-volatile ester oil chosen from purcellin oil (cetostearyl octanoate), isononyl isononanoate, C₁₂ to C₁₅ alkyl benzoate, 2-ethylhexyl palmitate, octyldodecyl neopentanoate, 2-octyldodecyl stearate, 2-octyldodecyl erucate, oleyl erucate, isostearyl isostearate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate, 2-octyldodecyl myristate and 2-diethylhexyl succinate.

Preferably, the composition according to the invention comprises at least one polar non-volatile oil chosen from neopentanoic acid esters, preferably octyldodecyl neopentanoate, and palmitic acid esters, preferably isopropyl palmitate.

A composition according to the invention may comprise a total content of non-volatile oil(s) ranging from 15% to 90% by weight, in particular from 25% to 80% by weight and preferably from 35% to 70% by weight relative to the total weight of the composition.

According to another embodiment, the polar non-volatile oil may be a fluoro oil.

The term *"fluoro oil"* means an oil comprising at least one fluorine atom.

The fluoro oils that may be used according to the invention may be chosen from fluorosilicone oils, fluoro polyethers and fluorosilicones as described in document EP-A-847 752, and perfluoro compounds.

According to the invention, the term "perfluoro compounds" is intended to mean compounds in which all the hydrogen atoms have been replaced with fluorine atoms.

According to one preferred embodiment, the fluoro oil according to the invention is chosen from perfluoro oils.

As examples of perfluoro oils that may be used in the invention, mention may be made of perfluorodecalins and perfluoroperhydrophenanthrenes.

According to one preferred embodiment, the fluoro oil is chosen from perfluoroperhydrophenanthrenes, and especially the Fiflow® products sold by the company Créations Couleurs. In particular, use may be made of the fluoro oil for which the INCI name is Perfluoroperhydrophenanthrene, sold under the reference Fiflow 220 by the company F2 Chemicals.

According to another embodiment, the polar non-volatile oil may be a silicone oil.

The non-volatile silicone oil that may be used in the invention may be chosen especially from silicone oils especially with a viscosity at 25°C of greater than or equal to 9 centistokes (cSt) (9 x 10⁻⁶ m²/s) and less than 800 000 cSt, preferably between 50 and 600 000 cSt and preferably between 100 and 500 000 cSt. The viscosity of this silicone may be measured according to standard ASTM D-445.

In particular, the non-volatile silicone oil may be chosen from:
- linear or branched non-volatile polydimethylsiloxanes (PDMS);
- polydimethylsiloxanes comprising alkyl, alkoxy or phenyl groups, which are pendent or at the end of the silicone chain, these groups each containing from 2 to 24 carbon atoms;
- phenyl silicone oils, in particular chosen from:
   - phenyl trimethicones, especially such as Phenyl trimethylsiloxy trisiloxane, sold especially under the reference Dow Corning 556 Cosmetic Grade Fluid;
   - phenyl dimethicones;
   - phenyl trimethylsiloxy diphenylsiloxanes;
   - diphenyl dimethicones;
   - diphenyl methyldiphenyl trisiloxanes;
   - 2-phenylethyl trimethylsiloxysilicates; and
   - trimethyl pentaphenyl trisiloxane, especially such as the silicone oils sold by Dow Corning under the reference PH-1555 HRI or Dow Corning 555 Cosmetic Fluid (chemical name: 1,3,5-trimethyl-1,1,3,5,5-pentaphenyl trisiloxane; INCI name: trimethyl pentaphenyl trisiloxane); and
- trimethyl siloxyphenyl dimethicones, especially such as the product sold under the reference Belsil PDM 1000 by the company Wacker.

Preferably, when the composition comprises a non-volatile silicone oil, it is chosen from non-phenylated silicone oils.

Preferably, the non-volatile silicone oil is a non-phenylated silicone oil having the INCI name Dimethicone.

In particular, the non-volatile non-phenylated silicone oil may be chosen from:
- linear or branched non-volatile polydimethylsiloxanes (PDMS);
- polydimethylsiloxanes comprising alkyl, alkoxy or phenyl groups, which are pendent or at the end of the silicone chain, these groups each containing from 2 to 24 carbon atoms.

A composition according to the invention may comprise a total content of non-volatile silicone oil(s) ranging from 15% to 90% by weight, in particular from 25% to 80% by weight and preferably from 35% to 70% by weight relative to the total weight of the composition.

Preferably, the non-volatile oil is chosen from:
- apolar hydrocarbon-based oils, preferably chosen from apolar hydrocarbon-based oils such as polybutenes, polyisobutenes, hydrogenated polyisobutenes, polydecenes and/or hydrogenated polydecenes, and mixtures thereof,
- polar hydrocarbon-based oils, preferably chosen from hydrocarbon-based oils from plants or of plant origin, ester oils, fatty alcohols containing from 12 to 26 carbon atoms and fatty acids containing from 12 to 26 carbon atoms, and mixtures thereof.
- silicone oils, preferably chosen from the non-phenylated silicone oils having the INCI name Dimethicone;
and mixtures thereof.

Preferably, if the composition contains any, the content of polar volatile oil(s), preferably hydrocarbon-based and/or silicone oil(s), ranges from 15% to 90% by weight, in particular from 25% to 80% by weight and preferably from 35% to 70% by weight relative to the total weight of the composition.

In accordance with a particularly advantageous embodiment, the composition according to the invention comprises at least one apolar non-volatile oil and at least one polar non-volatile oil.

### VOLATILE OILS

According to another embodiment, the composition according to the invention may comprise a volatile oil.

For the purposes of the invention, the term "volatile oil" means an oil that is capable of evaporating on contact with keratin materials in less than one hour, at room temperature and atmospheric pressure (760 mmHg). The volatile organic solvent(s) and volatile oils of the invention are volatile organic solvents and cosmetic oils that are liquid at room temperature, with a non-zero vapour pressure at room temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg), and more particularly ranging from 1.3 Pa to 1300 Pa (0.01 to 10 mmHg).

These oils may be hydrocarbon-based oils, silicone oils or fluoro oils, or mixtures thereof.

In particular, volatile oils that may be mentioned include volatile hydrocarbon-based oils and especially volatile hydrocarbon-based oils with a flash point of less than or equal to 80°C (the flash point is in particular measured according to ISO Standard 3679), such as hydrocarbon-based oils containing from 8 to 14 carbon atoms, and especially:
branched C₈-C₁₄ alkanes, for instance C₈-C₁₄ isoalkanes of petroleum origin (also known as isoparaffins), for instance isododecane (also known as 2,2,4,4,6-pentamethylheptane), isodecane, and, for example, the oils sold under the trade name Isopar or Permethyl,
   - linear alkanes, for instance n-dodecane (C12) and n-tetradecane (C14) sold by Sasol under the respective references Parafol 12-97 and Parafol 14-97, and also mixtures thereof, the undecane-tridecane mixture, the mixtures of n-undecane (C11) and of n-tridecane (C13) obtained in Examples 1 and 2 of patent application WO 2008/155 059 from the company Cognis, and mixtures thereof.

The volatile solvent is preferably chosen from volatile hydrocarbon-based oils containing from 8 to 14 carbon atoms, and mixtures thereof.

As other volatile hydrocarbon-based oils, and especially as volatile hydrocarbon-based oils with a flash point of less than or equal to 80°C, mention may also be made of ketones that are liquid at room temperature, such as methyl ethyl ketone or acetone; short-chain esters (containing from 3 to 8 carbon atoms in total) such as ethyl acetate, methyl acetate, propyl acetate or n-butyl acetate; ethers that are liquid at room temperature, such as diethyl ether, dimethyl ether or dichlorodiethyl ether; alcohols and especially linear or branched lower monoalcohols containing from 2 to 5 carbon atoms, such as ethanol, isopropanol or n-propanol.

A volatile hydrocarbon-based oil with a flash point of greater than 80°C that may be mentioned is isohexadecane.

According to a preferred embodiment, the composition according to the invention is free of volatile oil.

### PASTY FATTY SUBSTANCES

The composition according to the invention preferably comprises at least one pasty fatty substance.

For the purposes of the present invention, the term *"pasty fatty substance"* is intended to denote a lipophilic fatty compound that undergoes a reversible solid/liquid change of state, exhibiting anisotropic crystal organization in the solid state, and that comprises, at a temperature of 23°C, a liquid fraction and a solid fraction.

In other words, the starting melting point of the pasty fatty substance can be less than 23°C. The liquid fraction of the pasty fatty substance measured at 23°C can represent from 9% to 97% by weight of the pasty fatty substance. This liquid fraction at 23°C preferably represents between 15% and 85% and more preferably between 40% and 85% by weight.

Within the meaning of the invention, the melting point corresponds to the temperature of the most endothermic peak observed on thermal analysis (DSC) as described in Standard ISO 11357-3; 1999. The melting point of a pasty fatty substance may be measured using a differential scanning calorimeter (DSC), for example the calorimeter sold under the name MDSC 2920 by the company TA Instruments.

The measurement protocol is as follows:
A sample of 5 mg of pasty fatty substance placed in a crucible is subjected to a first temperature rise ranging from -20°C to 100°C, at a heating rate of 10°C/minute, is then cooled from 100°C to -20°C at a cooling rate of 10°C/minute and is finally subjected to a second temperature rise ranging from -20°C to 100°C at a heating rate of 5°C/minute. During the second temperature rise, the variation in the difference in power absorbed by the empty crucible and by the crucible containing the sample of pasty fatty substance is measured as a function of the temperature. The melting point of the pasty fatty substance is the value of the temperature corresponding to the tip of the peak of the curve representing the variation in the difference in power absorbed as a function of the temperature.

The liquid fraction by weight of the pasty fatty substance at 23°C is equal to the ratio of the heat of fusion consumed at 23°C to the heat of fusion of the pasty fatty substance.

The heat of fusion of the pasty fatty substance is the heat consumed by the substance in order to pass from the solid state to the liquid state. The pasty fatty substance is said to be in the solid state when all of its mass is in crystalline solid form. The pasty fatty substance is said to be in the liquid state when all of its mass is in liquid form.

The heat of fusion of the pasty fatty substance is equal to the area under the curve of the thermogram obtained using a differential scanning calorimeter (DSC), such as the calorimeter sold under the name MDSC 2920 by the company TA Instrument, with a temperature rise of 5°C or 10°C per minute, according to Standard ISO 11357-3; 1999.

The heat of fusion of the pasty fatty substance is the amount of energy required to make the pasty fatty substance change from the solid state to the liquid state. It is expressed in J/g.

The heat of fusion consumed at 23°C is the amount of energy absorbed by the sample to change from the solid state to the state that it has at 23°C, composed of a liquid fraction and a solid fraction.

The liquid fraction of the pasty fatty substance measured at 32°C preferably represents from 30% to 100% by weight of the pasty fatty substance, preferably from 50% to 100%, more preferably from 60% to 100% by weight of the pasty fatty substance. When the liquid fraction of the pasty fatty substance measured at 32°C is equal to 100%, the temperature of the end of the melting range of the pasty fatty substance is less than or equal to 32°C.

The liquid fraction of the pasty fatty substance measured at 32°C is equal to the ratio of the heat of fusion consumed at 32°C to the heat of fusion of the pasty fatty substance. The heat of fusion consumed at 32°C is calculated in the same way as the heat of fusion consumed at 23°C.

The pasty fatty substance may in particular be chosen from synthetic fatty substances and fatty substances of plant origin. A pasty fatty substance may be obtained by synthesis from starting materials of plant origin.

The pasty fatty substance may be chosen from:
- lanolin and derivatives thereof,
- petroleum jelly (also known as petrolatum),
- polyol ethers chosen from polyalkylene glycol pentaerythritol ethers, fatty alkyl ethers of a sugar, and mixtures thereof, polyethylene glycol pentaerythritol ether comprising 5 oxyethylene units (5 OE) (CTFA name: PPG-5 Pentaerythrityl Ether), polypropylene glycol pentaerythrityl ether comprising five oxypropylene (5 OP) units (CTFA name: PEG-5 Pentaerythrityl Ether) and mixtures thereof, and more especially the mixture PEG-5 Pentaerythrityl Ether, PPG-5 Pentaerythrityl Ether and soybean oil, sold under the name Lanolide by the company Vevy, which is a mixture in which the constituents are in a 46/46/8 weight ratio: 46% PEG-5 Pentaerythrityl Ether, 46% PPG-5 Pentaerythrityl Ether and 8% soybean oil,
- polymeric or non-polymeric silicone compounds,
- polymeric or non-polymeric fluorinated compounds,
- vinyl polymers, especially:
   ▪ olefin homopolymers and copolymers,
   ▪ hydrogenated diene homopolymers and copolymers,
   ▪ linear or branched oligomers, homopolymers or copolymers of alkyl (meth)acrylates preferably containing a C₈-C₃₀ alkyl group
   ▪ oligomers, which are homopolymers and copolymers of vinyl esters containing C₈-C₃₀ alkyl groups, and
   ▪ oligomers, which are homopolymers and copolymers of vinyl ethers containing C₈-C₃₀ alkyl groups
- liposoluble polyethers resulting from the polyetherification between one or more C₂-C₁₀₀ and preferably C₂-C₅₀ diols,
- esters,
- and/or mixtures thereof.

Among the fat-soluble polyethers that are particularly considered are copolymers of ethylene oxide and/or of propylene oxide with long-chain C₆-C₃₀ alkylene oxides, more preferably such that the weight ratio of the ethylene oxide and/or propylene oxide to alkylene oxides in the copolymer is from 5:95 to 70:30. In this family, mention will be made especially of copolymers such that the long-chain alkylene oxides are arranged in blocks having an average molecular weight from 1000 to 10 000, for example a polyoxyethylene/polydodecyl glycol block copolymer such as the ethers of dodecanediol (22 mol) and of polyethylene glycol (45 OE) sold under the brand name Elfacos ST9 by Akzo Nobel.

Among the esters, the following are especially considered:
- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, isostearic acid and 12-hydroxystearic acid, such as, for example, bis-diglyceryl polyacyladipate-2 sold under the reference Softisan® 649 by the company Sasol,
- vinyl ester homopolymers containing C₈-C₃₀ alkyl groups, such as polyvinyl laurate (sold especially under the reference Mexomer PP by the company Chimex),
- the arachidyl propionate sold under the brand name Waxenol 801 by Alzo,
- phytosterol esters,
- fatty acid triglycerides and derivatives thereof,
- pentaerythritol esters,
- esters of a diol dimer and of a diacid dimer, where appropriate esterified on their free alcohol or acid function(s) with acid or alcohol radicals, especially dimer dilinoleate esters; such esters may be chosen especially from the esters having the following INCI nomenclature: bis-behenyl/isostearyl/phytosteryl dimer dilinoleyl dimer dilinoleate (Plandool G), phytosteryl/isosteryl/cetyl/stearyl/behenyl dimer dilinoleate (Plandool H or Plandool S), and mixtures thereof,
- mango butter, such as the product sold under the reference Lipex 203 by the company AarhusKarlshamn,
- hydrogenated soybean oil, hydrogenated coconut oil, hydrogenated rape seed oil, mixtures of hydrogenated plant oils such as the mixture of hydrogenated soybean, coconut, palm and rapeseed plant oil, for example the mixture sold under the reference Akogel® by the company AarhusKarlshamn (INCI name: Hydrogenated plant Oil),
- shea butter, in particular the product for which the INCI name is Butyrospermum Parkii Butter, such as the product sold under the reference Sheasoft® by the company AarhusKarlshamn,
- and mixtures thereof.

According to a preferred embodiment, the pasty fatty substance is chosen from esters and in particular diglycerol esters, and mixtures thereof.

Among the pasty compounds, bis-behenyl/isostearyl/phytosteryl dimer dilinoleyl, bis(diglyceryl) poly(2-acyladipate), hydrogenated castor oil dimer dilinoleate, for example Risocast DA-L sold by Kokyu Alcohol Kogyo, and hydrogenated castor oil isostearate, for example Salacos HCIS (V-L) sold by Nisshin Oil, polyvinyl laurate, mango butter, shea butter, hydrogenated soybean oil, hydrogenated coconut oil and hydrogenated rapeseed oil, or a mixture thereof, will preferably be chosen.

Preferably, the composition according to the invention comprises a content of pasty fatty substances ranging from 0.1% to 50% by weight, in particular ranging from 1% to 45% by weight, and especially ranging from 5% to 40% by weight, relative to the total weight of the composition.

### HYDROPHOBIC SILICA AEROGELS

The composition according to the invention comprises at least silica aerogel particles.

Silica aerogels are porous materials obtained by replacing (by drying) the liquid component of a silica gel with air.

They are generally synthesized via a sol-gel process in a liquid medium and then dried, usually by extraction with a supercritical fluid, the one most commonly used being supercritical CO₂. This type of drying makes it possible to avoid shrinkage of the pores and of the material. The sol-gel process and the various drying operations are described in detail in Brinker C.J., and Scherer G.W., Sol-Gel Science: New York: Academic Press, 1990.

The hydrophobic silica aerogel particles used in the present invention have a specific surface area per unit of mass (S_{M}) ranging from 500 to 1500 m²/g, preferably from 600 to 1200 m²/g and better still from 600 to 800 m²/g, and a size expressed as the volume-average diameter (D[0.5]) ranging from 1 to 1500 µm, better still from 1 to 1000 µm, preferably from 1 to 100 µm, in particular from 1 to 30 µm, more preferably from 5 to 25 µm, better still from 5 to 20 µm and even better still from 5 to 15 µm.

According to one embodiment, the hydrophobic silica aerogel particles used in the present invention have a size, expressed as volume-average diameter (D[0.5]), ranging from 1 to 30 µm, preferably from 5 to 25 µm, better still from 5 to 20 µm and even better still from 5 to 15 µm.

The specific surface area per unit of mass can be determined by the nitrogen absorption method, known as the BET (Brunauer-Emmett-Teller) method, described in The Journal of the American Chemical Society, vol. 60, page 309, February 1938, which corresponds to international standard ISO 5794/1 (appendix D). The BET specific surface area corresponds to the total specific surface area of the particles under consideration.

The sizes of the silica aerogel particles may be measured by static light scattering using a commercial particle size analyser such as the MasterSizer 2000 machine from Malvern. The data are processed on the basis of the Mie scattering theory. This theory, which is exact for isotropic particles, makes it possible to determine, in the case of non-spherical particles, an *"effective"* particle diameter. This theory is described in particular in the publication by Van de Hulst, H.C., "Light Scattering by Small Particles", Chapters 9 and 10, Wiley, New York, 1957.

According to one advantageous embodiment, the hydrophobic silica aerogel particles used in the present invention have a specific surface area per unit of mass (S_{M}) ranging from 600 to 800 m²/g and a size expressed as the volume-average diameter (D[0.5]) ranging from 5 to 20 µm and even better still from 5 to 15 µm.

The silica aerogel particles used in the present invention may advantageously have a tapped density ρ ranging from 0.02 g/cm³ to 0.10 g/cm³, preferably from 0.03 g/cm³ to 0.08 g/cm³ and preferably from 0.05 g/cm³ to 0.08 g/cm³.

In the context of the present invention, this density, known as the tapped density, may be assessed according to the following protocol:
40 g of powder are poured into a measuring cylinder; the measuring cylinder is then placed on the Stav 2003 machine from Stampf Volumeter; the measuring cylinder is subsequently subjected to a series of 2500 tapping actions (this operation is repeated until the difference in volume between 2 consecutive tests is less than 2%); and then the final volume Vf of tapped powder is measured directly on the measuring cylinder. The tapped density is determined by the ratio m/Vf, in this instance 40/Vf (Vf being expressed in cm³ and m in g).

According to one preferred embodiment, the hydrophobic silica aerogel particles used in the present invention have a specific surface area per unit of volume S_{V} ranging from 5 to 60 m²/cm³, preferably from 10 to 50 m²/cm³ and better still from 15 to 40 m²/cm³.

The specific surface area per unit of volume is given by the relationship: S_{V} = S_{M} x ρ, where ρ is the tapped density, expressed in g/cm³, and S_{M} is the specific surface area per unit of mass, expressed in m²/g, as defined above.

Preferably, the hydrophobic silica aerogel particles according to the invention have an oil-absorbing capacity, measured at the wet point, ranging from 5 to 18 ml/g, preferably from 6 to 15 ml/g and better still from 8 to 12 ml/g.

The absorbing capacity measured at the wet point, noted Wp, corresponds to the amount of oil that needs to be added to 100 g of particles in order to obtain a homogeneous paste.

It is measured according to the "wet point" method or the method for determining the oil uptake of a powder described in standard NF T 30-022. It corresponds to the amount of oil adsorbed onto the available surface of the powder and/or absorbed by the powder by measurement of the wet point, described below:
An amount m = 2 g of powder is placed on a glass plate, and the oil (isononyl isononanoate) is then added dropwise. After addition of 4 to 5 drops of oil to the powder, mixing is carried out using a spatula, and addition of oil is continued until conglomerates of oil and powder have formed. From this point, the oil is added at the rate of one drop at a time and the mixture is subsequently triturated with the spatula. The addition of oil is stopped when a firm, smooth paste is obtained. This paste must be able to be spread on the glass plate without cracking or forming lumps. The volume Vs (expressed in ml) of oil used is then noted. The oil uptake corresponds to the ratio Vs/m.

The aerogels used according to the present invention are hydrophobic silica aerogels, preferably of silyl silica (INCI name: silica silylate).

The term "hydrophobic silica" is understood to mean any silica of which the surface is treated with silylating agents, for example with halogenated silanes, such as alkylchlorosilanes, siloxanes, in particular dimethylsiloxanes, such as hexamethyldisiloxane, or silazanes, so as to functionalize the OH groups with silyl Si-Rn groups, for example trimethylsilyl groups.

As regards the preparation of hydrophobic silica aerogel particles that have been surface-modified by silylation, reference may be made to document US 7 470 725.

Use will preferably be made of hydrophobic silica aerogel particles surface-modified with trimethylsilyl groups.

As hydrophobic silica aerogels that may be used in the invention, examples that may be mentioned include the aerogel sold under the name VM-2260 (INCI name: Silica silylate), by the company Dow Corning, the particles of which have an average size of about 1000 microns and a specific surface area per unit of mass ranging from 600 to 800 m²/g.

Mention may also be made of the aerogels sold by Cabot under the references Aerogel TLD 201, Aerogel OGD 201, Aerogel TLD 203, Enova® Aerogel MT 1100 and Enova Aerogel MT 1200.

Use will preferably be made of the aerogel sold under the name VM-2270 (INCI name: Silica silylate), by the company Dow Corning, the particles of which have an average size ranging from 5-15 microns and a specific surface area per unit of mass ranging from 600 to 800 m²/g.

Preferably, the hydrophobic silica aerogel particles are present in the composition according to the invention in an active material content ranging from 0.1% to 15% by weight and preferably from 0.1% to 10% by weight relative to the total weight of the composition.

Preferably, the hydrophobic silica aerogel particles are present in the composition according to the invention in an active material content ranging from 0.1% to 6% by weight and more preferably from 0.2% to 4% by weight relative to the total weight of the composition.

The hydrophobic silica aerogel particles may be used, especially in the context of the composition according to the invention, in a content range less than that conventionally used for the fillers conventionally used, especially in lip gloss compositions, such as nanosilica particles, such as the compound whose INCI name is Silica Dimethyl Silylate, sold especially under the reference Aerosil® R 972 by Evonik Degussa. Specifically, nanosilica particles are conventionally used in a weight content of between 2% and 7% by weight relative to the total weight of the composition.

This may prove to be advantageous in particular in the case of compositions for which it is important to be able to obtain a glossy deposit, in particular in the case of lip compositions, such as lip glosses (or sticks for solid compositions). Specifically, since fillers have a matting effect on the deposits obtained with the compositions, it is advantageous to be able to thicken and/or gel the formula sufficiently without thereby affecting the glossy nature of the deposit obtained, or doing so as little as possible.

Preferably, the composition according to the invention is free of nanometric-sized silica, and preferably the composition is free of hydrophobic-treated fumed silica, in particular such as the compound whose INCI name is Silica Dimethyl Silylate.

### VINYLPYRROLIDONE/OLEFIN COPOLYMER

As stated previously, the composition according to the invention comprises at least one vinylpyrrolidone/α-olefin copolymer. Advantageously, the α-olefin comprises from 2 to 40 carbon atoms and more particularly from 10 to 40 carbon atoms.

The polymer used in the context of the present invention may thus be defined as being a vinyl pyrrolidone derivative, more precisely either a copolymer of polyvinylpyrrolidone and of α-olefins, or a polyvinylpyrrolidone alkyl derivative.

These polymers are lipophilic.

They may especially comprise units represented by the following formula: 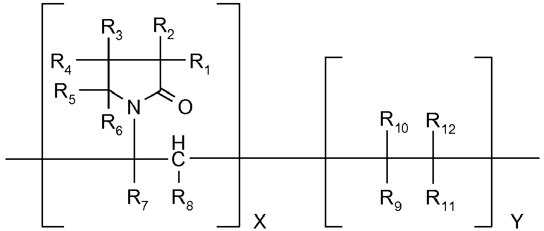 in which the radicals R₁ to R₁₂ represent, independently of each other, a saturated straight-chain or branched-chain C₁₀ to C₄₀ alkyl radical or a hydrogen atom, at least one of the said radicals R₁ to R₁₂ being other than a hydrogen atom.

The value y may be equal to 0; x is mandatorily non-zero.

Among the alkyl radicals comprising 10 to 40 carbon atoms, more particularly from 14 to 32 carbon atoms and preferentially 28 to 32 carbon atoms, mention may be made of pentadecyl, hexadecyl, heptadecyl, octadecyl, nonadecyl, eicosyl, docosyl and triacontyl radicals.

Advantageously, the weight-average molecular mass of the polymers according to the invention is between 5000 and 30 000 g/mol and in particular between 6000 and 20 000 g/mol; the coefficients x and y being chosen in function.

Preferably, y is non-zero. The radicals R₁ to R₉ and R₁₁ and R₁₂ preferably represent hydrogen. Preferably, R₁₀ comprises from 14 to 32 carbon atoms, and the ratio x/y is preferably between 1/5 and 5/1.

Among the polymers corresponding to this embodiment, mention may be made of the PVP/hexadecene copolymer (CTFA name) or the PVP/eicosene copolymer (CTFA name).

Among the commercial products, mention may be made of the products sold by the company GAF under the name Antaron V-216, which is a PVP/hexadecene copolymer comprising about 15-23% of pyrrolidone units, with a molecular weight of 7300; or under the name Antaron V-220, which is a PVP/eicosene copolymer which comprises about 20-28% by weight of pyrrolidone units and which has a mass-average molecular weight of 8600.

The polymer according to the invention has a consistency at room temperature that may be more or less viscous, depending on the alkyl chain length. It may thus be in liquid form with a viscosity of about 40 to 55 poises (4 to 5.5 Pa.s) at room temperature (25°C), in more pasty form or even in solid form close to the consistency of a wax.

Preferably, the vinylpyrrolidone/α-olefin polymer is chosen from the PVP/hexadecene copolymer (CTFA name) and the PVP/eicosene copolymer (CTFA name), or mixtures thereof.

Advantageously, the composition according to the invention comprises a content of copolymer of vinylpyrrolidone and of C₂-C₄₀ α-olefin of between 2% and 8% by weight relative to the weight of the composition and preferably between 2.5% and 7% by weight relative to the weight of the composition.

According to another embodiment, the composition is devoid of pasty fatty substances.

### HYDROCARBON-BASED RESIN

Preferably, the composition according to the invention comprises at least one hydrocarbon-based resin.

Preferably, the hydrocarbon-based resin (also known as a tackifying resin) has a number-average molecular weight of less than or equal to 10 000 g/mol, especially ranging from 250 to 5000 g/mol, better still less than or equal to 2000 g/mol and especially ranging from 250 to 2000 g/mol.

The number-average molecular weights (Mn) are determined by gel permeation liquid chromatography (THF solvent, calibration curve established with linear polystyrene standards, refractometric detector).

The resin of the composition according to the invention is advantageously what is known as a tackifying resin. Such resins are described especially in the Handbook of Pressure Sensitive Adhesive, edited by Donatas Satas, 3rd edition, 1989, pp. 609-619.

Preferably, the hydrocarbon-based resin is chosen from low molecular weight polymers that may be classified, according to the type of monomer they comprise, as:
- indene hydrocarbon-based resins, preferably such as resins derived from the polymerization in major proportion of indene monomer and in minor proportion of a monomer chosen from styrene, methylindene and methylstyrene, and mixtures thereof. These resins may optionally be hydrogenated. These resins may have a molecular weight ranging from 290 to 1150 g/mol.
   Examples of indene resins that may be mentioned include those sold under the reference Escorez 7105 by the company Exxon Chem., Nevchem 100 and Nevex 100 by the company Neville Chem., Norsolene S105 by the company Sartomer, Picco 6100 by the company Hercules and Resinall by the company Resinall Corp., or the hydrogenated indene/methylstyrene/styrene copolymers sold under the name "Regalite" by the company Eastman Chemical, in particular Regalite R 1100, Regalite R 1090, Regalite R-7100, Regalite R1010 Hydrocarbon Resin and Regalite R1125 Hydrocarbon Resin;
- aliphatic pentanediene resins such as those derived from the majority polymerization of the 1,3-pentanediene (trans- or cis-piperylene) monomer and of minor monomers chosen from isoprene, butene, 2-methyl-2-butene, pentene and 1,4-pentanediene, and mixtures thereof. These resins may have a molecular weight ranging from 1000 to 2500 g/mol.
   Such 1,3-pentanediene resins are sold, for example, under the references Piccotac 95 by the company Eastman Chemical, Escorez 1304 by the company Exxon Chemicals, Nevtac 100 by the company Neville Chem. or Wingtack 95 by the company Goodyear;
- mixed resins of pentanediene and of indene, which are derived from the polymerization of a mixture of pentanediene and indene monomers such as those described above, for instance the resins sold under the reference Escorez 2101 by the company Exxon Chemicals, Nevpene 9500 by the company Neville Chem., Hercotac 1148 by the company Hercules, Norsolene A 100 by the company Sartomer, and Wingtack 86, Wingtack Extra and Wingtack Plus by the company Goodyear;
- diene resins of cyclopentanediene dimers such as those derived from the polymerization of first monomers chosen from indene and styrene, and of second monomers chosen from cyclopentanediene dimers such as dicyclopentanediene, methyldicyclopentanediene and other pentanediene dimers, and mixtures thereof. These resins generally have a molecular weight ranging from 500 to 800 g/mol, for instance those sold under the reference Betaprene BR 100 by the company Arizona Chemical Co., Neville LX-685-125 and Neville LX-1000 by the company Neville Chem., Piccodiene 2215 by the company Hercules, Petro-Rez 200 by the company Lawter or Resinall 760 by the company Resinall Corp. ;
- diene resins of isoprene dimers such as terpenic resins derived from the polymerization of at least one monomer chosen from α-pinene, β-pinene and limonene, and mixtures thereof. These resins may have a molecular weight ranging from 300 to 2000 g/mol. Such resins are sold, for example, under the names Piccolyte A115 and S125 by Hercules or Zonarez 7100 or Zonatac 105 Lite by Arizona Chem.

Mention may also be made of certain modified resins such as hydrogenated resins, for instance those sold under the name Eastotac C6-C20 Polyolefin by the company Eastman Chemical Co., under the reference Escorez 5300 by the company Exxon Chemicals, or the resins Nevillac Hard or Nevroz sold by the company Neville Chem., the resins Piccofyn A-100, Piccotex 100 or Piccovar AP25 sold by the company Hercules or the resin SP-553 sold by the company Schenectady Chemical Co.

According to one preferred embodiment, the hydrocarbon-based resin is chosen from indene hydrocarbon-based resins, aliphatic pentadiene resins, mixed resins of pentanediene and of indene, diene resins of cyclopentanediene dimers and diene resins of isoprene dimers, or mixtures thereof.

Preferably, the composition comprises at least one compound chosen from hydrocarbon-based resins as described previously, especially indene hydrocarbon-based resins and aliphatic pentadiene resins, or mixtures thereof. According to one preferred embodiment, the hydrocarbon-based resin is chosen from indene hydrocarbon-based resins.

According to one preferred embodiment, the resin is chosen from hydrogenated indene/methylstyrene/styrene copolymers.

In particular, use may be made of hydrogenated indene/methylstyrene/styrene copolymers, such as those sold under the name Regalite by the company Eastman Chemical, such as Regalite R 1100, Regalite R 1090, Regalite R-7100, Regalite R 1010 Hydrocarbon Resin and Regalite R 1125 Hydrocarbon Resin.

Preferably, the hydrocarbon-based resin is present in the composition according to the invention in a content ranging from 1% to 45% by weight, preferably ranging from 3% to 30% by weight and more preferentially ranging from 5% to 25% by weight relative to the total weight of the composition.

### HYDROCARBON-BASED BLOCK COPOLYMER

Preferably, the composition according to the invention may comprise a hydrocarbon-based block copolymer, preferably a block copolymer that is soluble or dispersible in a liquid fatty phase as defined previously.

Such a compound is capable of thickening or gelling the organic phase of the composition. Preferably, the hydrocarbon-based block copolymer is an amorphous polymer, which means a polymer that does not have a crystalline form. Such a compound has film-forming properties, i.e. it is capable of forming a film when applied to the skin.

The hydrocarbon-based block copolymer may especially be a diblock, triblock, multiblock, radial or star copolymer, or mixtures thereof.

Such hydrocarbon-based block copolymers are described in patent application US-A-2002/005 562 and in patent US-A-5 221 534.

Preferably, the hydrocarbon-based block copolymer is obtained from at least one styrene monomer.

The copolymer may contain at least one block whose glass transition temperature is preferably less than 20°C, preferably less than or equal to 0°C, preferably less than or equal to -20°C and more preferably less than or equal to -40°C. The glass transition temperature of the said block may be between -150°C and 20°C and especially between -100°C and 0°C.

The hydrocarbon-based block copolymer present in the composition according to the invention is an amorphous copolymer formed by polymerization of an olefin. The olefin may especially be an elastomeric ethylenically unsaturated monomer.

Examples of olefins that may be mentioned include ethylenic carbide monomers, especially containing one or two ethylenic unsaturations and containing from 2 to 5 carbon atoms, such as ethylene, propylene, butadiene, isoprene or pentadiene.

Advantageously, the hydrocarbon-based block copolymer is an amorphous block copolymer of styrene and of an olefin.

Block copolymers comprising at least one styrene block and at least one block comprising units chosen from butadiene, ethylene, propylene, butylene and isoprene or a mixture thereof are especially preferred.

According to one preferred embodiment, the hydrocarbon-based block copolymer is hydrogenated to reduce the residual ethylenic unsaturations after the polymerization of the monomers.

In particular, the hydrocarbon-based block copolymer is a copolymer, optionally hydrogenated, containing styrene blocks and ethylene/C3-C4 alkylene blocks.

According to one preferred embodiment, the composition according to the invention comprises at least one diblock copolymer, which is preferably hydrogenated, preferably chosen from styrene-ethylene/propylene copolymers, styrene-ethylene/butadiene copolymers and styrene-ethylene/butylene copolymers. Diblock polymers are especially sold under the name Kraton® G1701E by the company Kraton Polymers.

According to another preferred embodiment, the composition according to the invention comprises at least one triblock copolymer, which is preferably hydrogenated, preferably chosen from styrene-ethylene/propylene-styrene copolymers, styrene-ethylene/butadiene-styrene copolymers, styrene-isoprene-styrene copolymers and styrene-butadiene-styrene copolymers. Triblock polymers are especially sold under the names Kraton® G1650, Kraton® G1652, Kraton® D1101, Kraton® D1102 and Kraton® D1160 by the company Kraton Polymers.

According to one embodiment of the present invention, the hydrocarbon-based block copolymer is a styrene-ethylene/butylene-styrene triblock copolymer.

According to one preferred embodiment of the invention, it is especially possible to use a mixture of a styrene-butylene/ethylene-styrene triblock copolymer and of a styrene-ethylene/butylene diblock copolymer, especially the products sold under the name Kraton® G1657M by the company Kraton Polymers.

According to another preferred embodiment, the composition according to the invention comprises a mixture of styrene-butylene/ethylene-styrene hydrogenated triblock copolymer and of ethylene-propylene-styrene hydrogenated star polymer, such a mixture possibly being especially in isododecane or in another oil. Such mixtures are sold, for example, by the company Penreco under the trade names Versagel® M5960 and Versagel® M5670.

Advantageously, a diblock copolymer such as those described previously is used as polymeric gelling agent, in particular a styrene-ethylene/propylene diblock copolymer or a mixture of diblock and triblock copolymers, as described previously.

The hydrocarbon-based block copolymer (or the mixture of hydrocarbon-based block copolymers) may be present in a content ranging from 0.1% to 15% by weight and preferably ranging from 0.5% to 10% by weight relative to the total weight of the composition.

Preferably, when the composition is in solid form, the hydrocarbon-based block copolymer is present in the composition according to the invention in a content ranging from 0.1% to 10% by weight and more preferentially ranging from 1% to 5% by weight relative to the total weight of the composition.

Preferably, when the composition is in liquid form, the hydrocarbon-based block copolymer is present in the composition according to the invention in a content ranging from 3% to 15% by weight and more preferentially ranging from 5% to 10% by weight relative to the total weight of the composition.

Preferably, the weight ratio of the hydrocarbon-based resin to the hydrocarbon-based block copolymer is between 1 and 10.

More preferably, the weight ratio of the hydrocarbon-based resin to the hydrocarbon-based block copolymer is between 1 and 8.

More preferably, the weight ratio of the hydrocarbon-based resin to the hydrocarbon-based block copolymer is between 1 and 5 and preferably between 1 and 3.

### DEXTRIN ESTER

The composition according to the invention may also comprise at least one preferably C₁₂-C₂₄ and in particular C₁₄-C₁₈ fatty acid ester of dextrin.

Preferably, the dextrin ester is an ester of dextrin and of a C₁₂-C₁₈ and in particular C₁₄-C₁₈ fatty acid.

Preferably, the dextrin ester is chosen from dextrin myristate and/or dextrin palmitate, and mixtures thereof.

According to a particular embodiment, the dextrin ester is dextrin myristate, especially such as the product sold under the name Rheopearl MKL-2 by the company Chiba Flour.

According to a preferred embodiment, the dextrin ester is dextrin palmitate. This product may be chosen, for example, from those sold under the names Rheopearl TL® and Rheopearl KL® by the company Chiba Flour.

The composition according to the invention may particularly preferably comprise between 0.1% and 10% by weight and preferably between 0.5% and 5% by total weight of dextrin ester(s) relative to the total weight of the composition.

The composition according to the invention may particularly preferably comprise between 0.1% and 10% by weight and preferably between 0.5% and 5% by total weight of dextrin palmitate relative to the total weight of the composition, especially such as the products sold under the names Rheopearl TL® and Rheopearl KL® by the company Chiba Flour.

### C₂-C₆ CARBOXYLIC ACID ESTER OF SUCROSE

A composition according to the invention may also comprise at least one C₂-C₆ carboxylic acid ester of sucrose.

More particularly, this C₂-C₆ carboxylic acid ester of sucrose is chosen from mixed esters of acetic acid, isobutyric acid and sucrose, and in particular sucrose diacetate hexakis(2-methylpropanoate), such as the product sold under the name Sustane SAIB Food Grade Kosher by the company Eastman Chemical (INCI name: sucrose acetate isobutyrate).

Advantageously, a composition of the invention may comprise from 1% to 15% by weight and preferably from 3% to 10% by weight of C₂-C₆ carboxylic acid ester(s) of sucrose relative to the total weight of the said composition.

### Moisturizer

The composition according to the invention may comprise at least one moisturizer. Preferably, the moisturizer may be chosen from: sorbitol, polyhydric alcohols, preferably of C₂-C₈ and more preferably of C₃-C₆, preferably such as glycerol, propylene glycol, 1,3-butylene glycol, dipropylene glycol, diglycerol and glycerol, and mixtures thereof.

According to one particular mode, the moisturizer is glycerol.

The moisturizer is preferably present in the fatty phase in a content of between 0.1% and 10% by weight relative to the total weight of the composition.

### DYESTUFFS

The composition according to the invention preferably comprises at least one dyestuff (also known as a colouring agent), which may be chosen from water-soluble or liposoluble dyes, pigments and nacres, and mixtures thereof.

The composition according to the invention may also comprise one or more dyestuffs chosen from water-soluble dyes and pulverulent dyestuffs, for instance pigments, nacres and glitter flakes that are well known to those skilled in the art.

In a particularly preferred manner, the composition according to the invention comprises at least one dyestuff chosen from pigments and/or nacres.

In particular, a composition according to the invention must be sufficiently stable for the pigments and/or nacres - when they are present in the composition - to remain in suspension and not to sediment out.

The dyestuffs may be present in the composition in a content ranging from 0.01% to 20% by weight, relative to the weight of the composition, preferably from 0.1% to 15% by weight.

The term "pigments" should be understood as meaning white or coloured, mineral or organic particles that are insoluble in an aqueous solution, which are intended to colour and/or opacify the resulting film.

The pigments may be present in a proportion of from 0.01% to 20% by weight, especially from 0.1% to 15% by weight and in particular from 0.2% to 10% by weight, relative to the total weight of the cosmetic composition.

As mineral pigments that may be used in the invention, mention may be made of titanium oxide, zirconium oxide or cerium oxide, and also zinc oxide, iron oxide or chromium oxide, ferric blue, manganese violet, ultramarine blue and chromium hydrate.

The pigment may also be a pigment having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

The dyestuff may also comprise a pigment with a structure that may be, for example, of silica microsphere type containing iron oxide. An example of a pigment having this structure is the product sold by the company Miyoshi under the reference PC Ball PC-LL-100 P, this pigment consisting of silica microspheres containing yellow iron oxide.

Among the organic pigments that may be used in the invention, mention may be made of carbon black, pigments of D&C type, lakes based on cochineal carmine or on barium, strontium, calcium or aluminium, or alternatively the diketopyrrolopyrroles (DPPs) described in documents EP-A-542 669, EP-A-787 730, EP-A-787 731 and WO-A-96/08537.

The terms "nacres" should be understood as meaning coloured particles of any form, which may or may not be iridescent, especially produced by certain molluscs in their shell, or alternatively synthesized, and which have a colour effect via optical interference.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles at the surface of which are superposed at least two successive layers of metal oxides and/or of organic dyestuffs.

Examples of nacres that may also be mentioned include natural mica coated with titanium oxide, with iron oxide, with natural pigment or with bismuth oxychloride.

Among the commercially available nacres that may be mentioned are the nacres Timica, Flamenco and Duochrome (on mica base) sold by the company Engelhard, the Timiron nacres sold by the company Merck, the Prestige nacres on mica base sold by the company Eckart and the Sunshine nacres on synthetic mica base sold by the company Sun Chemical.

The nacres may more particularly have a yellow, pink, red, bronze, orangey, brown, gold and/or coppery colour or tint.

As illustrations of nacres that may be used in the context of the present invention, mention may be made especially of the gold-coloured nacres sold especially by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold especially by the company Merck under the name Bronze fine (17384) (Colorona) and Bronze (17353) (Colorona) and by the company Engelhard under the name Super bronze (Cloisonne); the orange nacres sold especially by the company Engelhard under the name Orange 363C (Cloisonne) and Orange MCR 101 (Cosmica) and by the company Merck under the name Passion orange (Colorona) and Matte orange (17449) (Microna); the brown nacres sold especially by the company Engelhard under the name Nu-antique copper 340XB (Cloisonne) and Brown CL4509 (Chromalite); the nacres with a copper tint sold especially by the company Engelhard under the name Copper 340A (Timica); the nacres with a red tint sold especially by the company Merck under the name Sienna fine (17386) (Colorona); the nacres with a yellow tint sold especially by the company Engelhard under the name Yellow (4502) (Chromalite); the red nacres with a gold tint sold especially by the company Engelhard under the name Sunstone G012 (Gemtone); the pink nacres sold especially by the company Engelhard under the name Tan opale G005 (Gemtone); the black nacres with a gold tint sold especially by the company Engelhard under the name Nu antique bronze 240 AB (Timica), the blue nacres sold especially by the company Merck under the name Matte blue (17433) (Microna), the white nacres with a silvery tint sold especially by the company Merck under the name Xirona Silver, and the golden-green pink-orange nacres sold especially by the company Merck under the name Indian summer (Xirona), and mixtures thereof.

The pigments and nacres may be present in the composition in a total content ranging from 0.1% to 20% by weight, relative to the weight of the composition, preferably from 0.5% to 15% by weight.

The term "dyes" should be understood as meaning compounds that are generally organic, which are soluble in fatty substances such as oils or in an aqueous-alcoholic phase.

The cosmetic composition according to the invention may also comprise water-soluble or liposoluble dyes. The liposoluble dyes are, for example, Sudan red, DC Red 17, DC Green 6, β-carotene, Sudan brown, DC Yellow 11, DC Violet 2, DC Orange 5 and quinoline yellow. The water-soluble dyes are, for example, beetroot juice or methylene blue.

The cosmetic composition according to the invention may also contain at least one material with a specific optical effect as dyestuff.

This effect is different from a simple conventional hue effect, i.e. a unified and stabilized effect as produced by standard dyestuffs, for instance monochromatic pigments. For the purposes of the invention, the term "stabilized" means lacking an effect of variability of the colour as a function of the angle of observation or alternatively in response to a temperature change.

For example, this material may be chosen from particles with a metallic glint, goniochromatic colouring agents, diffractive pigments, thermochromic agents, optical brighteners, and also fibres, especially interference fibres. Needless to say, these various materials may be combined so as to afford the simultaneous manifestation of two effects, or even of a novel effect in accordance with the invention.

### PULVERENT PHASE

The composition according to the invention preferably comprises at least one pulverulent phase.

Preferably, the pulverulent phase represents between 0.1% and 25% by weight, preferably between 0.1% and 20% by weight and preferably between 0.5% and 20% by weight relative to the total weight of the composition.

Preferably, the pulverulent phase represents between 1% and 20% by weight relative to the total weight of the composition.

Preferably, the pulverulent phase according to the invention comprises at least silica aerogel particles, optionally at least one additional filler and/or at least one dyestuff chosen from nacres and/or pigments, and mixtures thereof.

### ADDITIONAL FILLERS

A composition according to the invention may contain at least one or more additional filler(s).

The term "fillers" should be understood as meaning colourless or white, mineral or synthetic particles of any shape, which are insoluble in the medium of the composition, irrespective of the temperature at which the composition is manufactured. These fillers serve especially to modify the rheology or the texture of the composition.

The additional fillers may be mineral or organic and of any shape, platelet-shaped, spherical or oblong, irrespective of the crystallographic form (for example lamellar, cubic, hexagonal, orthorhombic, etc.).

Preferably, the said additional filler(s) are chosen from talc, mica, silica, kaolin, bentone, polyamide (Nylon®) powder (Orgasol® from Atochem), poly-β-alanine powder and polyethylene powder, tetrafluoroethylene polymer (Teflon®) powder, lauroyllysine, starch, boron nitride, hollow polymer microspheres such as polyvinylidene chloride/acrylonitrile microspheres, for instance Expancel® (Nobel Industrie), acrylic acid copolymer microspheres (Polytrap® from the company Dow Corning) and silicone resin microbeads (for example Tospearls® from Toshiba), precipitated calcium carbonate, magnesium carbonate, magnesium hydrogen carbonate, hydroxyapatite, hollow silica microspheres (Silica Beads® from Maprecos), elastomeric polyorganosiloxane particles, glass or ceramic microcapsules, and metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate.

Preferably, the said additional filler(s) are chosen from talc, mica, silica, kaolin, bentone, polyamide (Nylon®) powder (Orgasol® from Atochem), poly-β-alanine powder and polyethylene powder, tetrafluoroethylene polymer (Teflon®) powder, lauroyllysine, starch, boron nitride, hollow polymer microspheres such as polyvinylidene chloride/acrylonitrile microspheres, for instance Expancel® (Nobel Industrie), acrylic acid copolymer microspheres (Polytrap® from the company Dow Corning) and silicone resin microbeads (for example Tospearls® from Toshiba), precipitated calcium carbonate, magnesium carbonate, magnesium hydrogen carbonate, hydroxyapatite, hollow silica microspheres (Silica Beads® from Maprecos), elastomeric polyorganosiloxane particles, glass or ceramic microcapsules, and metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate.

The composition according to the invention may also comprise, as additional filler, particles comprising a copolymer, the said copolymer comprising trimethylol hexyl lactone. In particular, it may be a copolymer of hexamethylene diisocyanate/trimethylol hexyl lactone. Such particles are especially commercially available, for example, under the name Plastic Powder D-400® or Plastic Powder D-800® from the company Toshiki.

Preferably, the composition contains between 0.1% and 15% by total weight and in particular between 0.1% and 10% by total weight of additional fillers relative to the total weight of the composition.

### ADDITIVES

A composition according to the invention may furthermore comprise any ingredient conventionally used as additive in cosmetics and dermatology.

These additives are advantageously chosen from antioxidants, thickeners, sweeteners, acidifying or basifying agents, preserving agents such as glycols, film-forming polymers other than those mentioned, and hydrocarbon-based block copolymers, and mixtures thereof.

Needless to say, those skilled in the art will take care to select this or these optional additional compound(s), and/or the amount thereof, such that the advantageous properties of the composition according to the invention are not, or are not substantially, adversely affected by the envisaged addition.

The composition according to the invention is in liquid form.

The term "liquid" means a fluid texture, i.e. which may especially be in creamy or pasty form.

The term "fluid" especially means a composition that is not solid at room temperature (20-25°C) and whose viscosity it is possible to measure.

The compositions according to the invention may especially be in gloss form, intended for making up and/or caring for the skin or the lips.

### Protocol for measuring the viscosity:

The viscosity measurement is generally performed at 25°C, using a Rheomat RM 180 viscometer equipped with a No. 4 spindle, the measurement being performed after 10 minutes of rotation of the spindle in the composition (after which time stabilization of the viscosity and of the spin speed of the spindle are observed), at a shear rate of 200 rpm.

Preferably, the composition has at 25°C a viscosity of between 1 and 25 Pa.s and preferably between 2 and 20 Pa.s.

The terms "between" and "ranging from" should be understood as including the limits.

The example that follows is given as an illustration, without any limiting nature.

Unless otherwise mentioned, the values in the example below are expressed as weight percentages relative to the total weight of the composition.

### EXAMPLE:

The liquid lipstick composition that follows is prepared (contents expressed as weight of active material):

| | |
|---|---|
| Castor oil (*Ricinus communis* seed oil) | 15 |
| Trihydroxystearin (Thixcin R from Elementis Specialties) | 2.5 |
| Bis-diglyceryl- poly-acyladipate-2 (Softisan 649 from Sasol) | 13.5 |
| Diisostearyl malate | 7.3 |
| Pentaerythrityl tetraisostearate | 12.2 |
| Tridecyl trimellitate | 8 |
| C18-36 acid triglyceride (DUB TGI 24 from Stearinerie Dubois) | 14.8 |
| Hydroxystearic acid | 1.7 |
| Silica silylate (hydrophobic silica aerogel; Aerogel VM2270 from Dow Corning) | 0.5 |
| Mica | 0.8 |
| Polyethylene wax (Asensa SC 211 from Honeywell) | 1 |
| Polybutene (Indopol H-100 from INEOS) | 15 |
| Vinylpyrrolidone/hexadecene copolymer (Antaron V216 from ISP) | 5 |
| Pentylene glycol | 1 |
| Pigments and nacres | qs |

### Preparation process

In a first stage, the pigments were ground in a three-roll mill in part of the mixture of non-volatile oils present.

The rest of the non-volatile oils and the other fatty substances (waxes, pasty substances) were then mixed in a heating pan and the mixture was brought to a temperature of 100°C with stirring using a Rayneri blender until a homogeneous mixture was obtained.

The ground pigmentary material was then incorporated into the said mixture and stirring was continued until the mixture was homogeneous.

The silica aerogel, the mica and the nacres were then added, and the resulting mixture was stirred.

The mixture was cooled with stirring to a temperature of less than or equal to 60°C.

Finally, the composition was poured into heating sachets and then placed at room temperature for 24 hours.

### Evaluation of the compositions

Viscosity: The composition has a viscosity of 5.9 Pa.s at 25°C according to the protocol described previously.

Stability: the stability of the compositions is evaluated by storing the composition for 24 hours at room temperature and by observing whether separation of the oily phase and/or sedimentation of the pigments and/or nacres takes place.

The stability of the compositions was also evaluated after centrifugation at a speed of 450×g for 10 minutes.

The composition is stable for 24 hours at room temperature and under centrifugation.

Moreover, the composition was easily applied on the lips and formed a thin, homogeneous, non tacky, glossy and comfortable film.

## Claims

1. Liquid cosmetic composition, preferably for making up and/or caring for the lips and/or the skin, comprising, in a physiologically acceptable medium, at least one fatty phase containing:
- at least one non-volatile oil,
- at least one linear C₁₈-C₂₄ hydroxylated fatty acid, preferably 12-hydroxystearic acid,
- at least one additional wax other than the said linear C₁₈-C₂₄ hydroxylated fatty acid,
- at least one copolymer of vinylpyrrolidone and of at least one C₂-C₄₀ α-olefin,
- at least hydrophobic silica aerogel particles,
- the said composition optionally comprising water at less than 5% by weight relative to the total weight of the composition, and preferably being anhydrous.

2. Composition according to the preceding claim, **characterized in that** it comprises a content of linear C₁₈-C₂₄ hydroxylated fatty acid(s) ranging from 0.1% to 5% by weight, better still preferably from 0.1% to 4% by weight and preferably from 0.5% to 3% by weight relative to the total weight of the composition.

3. Composition according to any one of the preceding claims, **characterized in that** the additional wax(es) are chosen from apolar waxes; polar waxes such as ester waxes, alcohol waxes or silicone waxes; or mixtures thereof, and preferably from the waxes corresponding to the total esters of a saturated, optionally hydroxylated C₁₆-C₃₀ carboxylic acid with glycerol, such as trihydroxystearin; beeswax; synthetic beeswax; polyglycerolated beeswax; carnauba wax; candelilla wax; oxypropylenated lanolin wax; rice bran wax; ouricury wax; esparto grass wax; cork fibre wax; sugar cane wax; Japan wax; sumach wax; montan wax; orange wax; laurel wax; hydrogenated jojoba wax, alone or as a mixture; from polyethylene waxes, polymethylene waxes, ozokerite and microcrystalline waxes, alone or as mixtures.

4. Composition according to the preceding claim, **characterized in that** the said additional wax is chosen from waxes with a melting point of at least 60°C.

5. Composition according to any one of the preceding claims, **characterized in that** the content of additional wax(es) is between 0.1% and 10% by weight, preferably between 0.5% and 7% by weight and even more particularly between 0.5% and 5% by weight, relative to the total weight of the composition.

6. Composition according to any one of the preceding claims, **characterized in that** the copolymer of vinylpyrrolidone and of a C2-C40 α-olefin is preferably chosen from copolymers of vinylpyrrolidone and hexadecene and of vinylpyrrolidone and eicosene.

7. Composition according to any one of the preceding claims, **characterized in that** the content of copolymer of vinylpyrrolidone and of a C₂-C₄₀ olefin is between 2% and 8% by weight relative to the weight of the composition and preferably between 2.5% and 7% by weight relative to the weight of the composition.

8. Composition according to any one of the preceding claims, **characterized in that** the non-volatile oil is chosen from apolar hydrocarbon-based oils, preferably chosen from liquid paraffin or derivatives thereof, squalane, isoeicosane, naphthalene oil, polybutylenes, hydrogenated or non-hydrogenated polyisobutenes, decene/butene copolymers, polybutene/polyisobutene copolymers, hydrogenated or non-hydrogenated polydecenes, and mixtures thereof, and more particularly from polybutenes, polyisobutenes hydrogenated polyisobutenes, polydecenes and/or hydrogenated polydecenes, and mixtures thereof.

9. Composition according to any one of the preceding claims, **characterized in that** it comprises a content of apolar non-volatile oil(s) of between 5% and 60% by weight and in particular between 10% and 45% by weight, relative to the total weight of the composition.

10. Composition according to any one of the preceding claims, **characterized in that** the non-volatile oil is chosen from polar hydrocarbon-based, silicone or fluoro oils and preferably from hydrocarbon-based plant oils or oils of plant origin, ester oils, fatty alcohols containing from 12 to 26 carbon atoms, fatty acids containing from 12 to 26 carbon atoms and silicone oils, preferably chosen from the non-phenylated silicone oils whose INCI name is Dimethicone, and mixtures thereof.

11. Composition according to any one of the preceding claims, **characterized in that** it comprises a content of polar non-volatile oil(s) of between 15% and 90% by weight, in particular between 25% and 80% by weight and preferably between 35% and 70% by weight, relative to the total weight of the composition.

12. Composition according to any one of the preceding claims, **characterized in that** it comprises at least one dyestuff chosen from pigments and/or nacres, preferably in a total content of between 0.1% and 20% and preferably between 0.5% and 15% by weight relative to the total weight of the composition.

13. Composition according to any one of the preceding claims, **characterized in that** the hydrophobic silica aerogel particles have a specific surface area per unit of mass (S_{M}) ranging from 500 to 1500 m²/g, preferably from 600 to 1200 m²/g and better still from 600 to 800 m²/g, and a size expressed as the volume-average diameter (D[0.5]) ranging from 1 to 1500 µm, preferably from 1 to 1000 µm, more preferentially from 1 to 100 µm, in particular from 1 to 30 µm, more preferably from 5 to 25 µm, better still from 5 to 20 µm and even better still from 5 to 15 µm.

14. Composition according to any one of the preceding claims, **characterized in that** the hydrophobic aerogel particles have an oil absorption capacity, measured at the wet point in accordance with the method defined in the description, ranging from 5 to 18 ml/g, preferably from 6 to 15 ml/g and better still from 8 to 12 ml/g of particles.

15. Composition according to any one of the preceding claims, **characterized in that** the hydrophobic aerogel particles have a tapped density measured in accordance with the method defined in the description ranging from 0.02 g/cm³ to 0.10 g/cm³ and preferably from 0.03 g/cm³ to 0.08 g/cm³.

16. Composition according to any one of the preceding claims, **characterized in that** the hydrophobic silica aerogel particles are hydrophobic silica aerogel particles that are surface-modified with trimethylsilyl groups, preferably hydrophobic silica aerogel particles having the INCI name Silica silylate.

17. Composition according to any one of the preceding claims, **characterized in that** the hydrophobic aerogel particles are present in an active material content ranging from 0.1% to 15% by weight, preferably from 0.1% to 10% by weight, preferably from 0.1% to 6% by weight and more preferably from 0.2% to 4% by weight, relative to the total weight of the composition.

18. Cosmetic process for making up and/or caring for the skin and/or the lips, comprising the application to the skin and/or the lips of a composition as defined in any one of the preceding claims.

## Patentansprüche

1. Flüssige kosmetische Zusammensetzung, vorzugsweise zum Schminken und/oder Pflegen der Lippen und/ oder der Haut, die in einem physiologisch unbedenklichen Medium mindestens eine Fettphase umfasst, welche
- mindestens ein nichtflüchtiges Öl,
- mindestens eine lineare hydroxylierte C₁₈-C₂₄-Fettsäure, vorzugsweise 12-Hydroxystearinsäure,
- mindestens ein zusätzliches Wachs, das von der linearen hydroxylierten C₁₈-C₂₄-Fettsäure verschieden ist,
- mindestens ein Copolymer von Vinylpyrrolidon und mindestens einem C₂-C₄₀-α-Olefin,
- mindestens hydrophobe Siliciumdioxid-Aerogel-Teilchen
enthält, wobei die Zusammensetzung gegebenenfalls Wasser in einer Menge von weniger als 5 Gew.-%, bezogen auf das Gesamtgewicht der Zusammensetzung, umfasst und vorzugsweise wasserfrei ist.

2. Zusammensetzung nach dem vorhergehenden Anspruch, **dadurch gekennzeichnet, dass** sie einen Gehalt von linearer hydroxylierter C₁₈-C₂₄-Fettsäure bzw. linearen hydroxylierten C₁₈-C₂₄-Fettsäuren im Bereich von 0,1 bis 5 Gew.-%, noch weiter bevorzugt von 0,1 bis 4 Gew.-% und vorzugsweise 0,5 bis 3 Gew.-%, bezogen auf das Gesamtgewicht der Zusammensetzung, umfasst.

3. Zusammensetzung nach einem der vorhergehenden Ansprüche, **dadurch gekennzeichnet, dass** das zusätzliche Wachs bzw. die zusätzlichen Wachse aus unpolaren Wachsen; polaren Wachsen wie Esterwachsen, Alkoholwachsen oder Silikonwachsen oder Mischungen davon und vorzugsweise aus den Wachsen, die den Vollestern einer gesättigten, gegebenenfalls hydroxylierten C₁₆-C₃₀-Carbonsäure mit Glycerin entsprechen, wie Trihydroxystearin; Bienenwachs; synthetischem Bienenwachs; polyglyceriniertem Bienenwachs; Carnaubawachs; Candelillawachs; oxypropyleniertem Lanolinwachs; Reiskleiewachs; Ouricury-Wachs; Espartograswachs; Korkfaserwachs; Zuckerrohrwachs; Japanwachs; Sumachwachs; Montanwachs; Orangenwachs; Lorbeerwachs; hydriertem Jojobawachs, alleine oder als Mischung; aus Polyethylenwachsen, Polymethylenwachsen, Ozokerit und mikrokristallinen Wachsen, alleine oder als Mischungen, ausgewählt ist bzw. sind.

4. Zusammensetzung nach dem vorhergehenden Anspruch, **dadurch gekennzeichnet, dass** das zusätzliche Wachs aus Wachsen mit einem Schmelzpunkt von mindestens 60 °C ausgewählt ist.

5. Zusammensetzung nach einem der vorhergehenden Ansprüche, **dadurch gekennzeichnet, dass** der Gehalt an zusätzlichem Wachs bzw. zusätzlichen Wachsen zwischen 0,1 und 10 Gew.-%, vorzugsweise zwischen 0,5 und 7 Gew. -% und noch spezieller zwischen 0,5 und 5 Gew.-%, bezogen auf das Gesamtgewicht der Zusammensetzung, liegt.

6. Zusammensetzung nach einem der vorhergehenden Ansprüche, **dadurch gekennzeichnet, dass** das Copolymer von Vinylpyrrolidon und einem C₂-C₄₀-α-Olefin vorzugsweise aus Copolymeren von Vinylpyrrolidon und Hexadecen und Vinylpyrrolidon und Eicosen ausgewählt ist.

7. Zusammensetzung nach einem der vorhergehenden Ansprüche, **dadurch gekennzeichnet, dass** der Gehalt an Copolymer von Vinylpyrrolidon und einem C₂-C₄₀-Olefin zwischen 2 und 8 Gew.-%, bezogen auf das Gewicht der Zusammensetzung, und vorzugsweise zwischen 2,5 und 7 Gew.-%, bezogen auf das Gewicht der Zusammensetzung, liegt.

8. Zusammensetzung nach einem der vorhergehenden Ansprüche, **dadurch gekennzeichnet, dass** das nichtflüchtige Öl aus unpolaren Ölen auf Kohlenwasserstoffbasis ausgewählt ist, die vorzugsweise aus Flüssigparaffin und Derivaten davon, Squalan, Isoeicosan, Naphthalinöl, Polybutylenen, hydrierten oder nicht hydrierten Polyisobutenen, Decen/Buten-Copolymeren, Polybuten/Polyisobuten-Copolymeren, hydrierten oder nicht hydrierten Polydecenen und Mischungen davon und spezieller aus Polybutenen, Polyisobutenen, hydrierten Polyisobutenen, Polydecenen und/oder hydrierten Polydecenen und Mischungen davon ausgewählt sind.

9. Zusammensetzung nach einem der vorhergehenden Ansprüche, **dadurch gekennzeichnet, dass** sie einen Gehalt an unpolarem nichtflüchtigem Öl bzw. unpolaren nichtflüchtigen Ölen zwischen 5 und 60 Gew.-% und insbesondere zwischen 10 und 45 Gew.-%, bezogen auf das Gesamtgewicht der Zusammensetzung, umfasst.

10. Zusammensetzung nach einem der vorhergehenden Ansprüche, **dadurch gekennzeichnet, dass** das nichtflüchtige Öl aus polaren Ölen auf Kohlenwasserstoffbasis, Silikonölen oder Fluorölen und vorzugsweise aus auf Kohlenwasserstoff basierenden Pflanzenölen oder Ölen pflanzlichen Ursprungs, Esterölen, Fettalkoholen mit 12 bis 26 Kohlenstoffatomen, Fettsäuren mit 12 bis 26 Kohlenstoffätomen und Silikonölen, die vorzugsweise aus den nichtphenylierten Silikonölen mit dem INCI-Namen Dimethicone ausgewählt sind, und Mischungen davon ausgewählt ist.

11. Zusammensetzung nach einem der vorhergehenden Ansprüche, **dadurch gekennzeichnet, dass** sie einen Gehalt an polarem nichtflüchtigem Öl bzw. polaren nichtflüchtigen Ölen zwischen 15 und 90 Gew.-%, insbesondere zwischen 25 und 80 Gew.-% und vorzugsweise zwischen 35 und 70 Gew.-%, bezogen auf das Gesamtgewicht der Zusammensetzung, umfasst.

12. Zusammensetzung nach einem der vorhergehenden Ansprüche, **dadurch gekennzeichnet, dass** sie mindestens einen Farbstoff, der aus Pigmenten und/oder Perlmutten ausgewählt ist, umfasst, vorzugsweise in einem Gesamtgehalt zwischen 0,1 und 20 Gew.-% und vorzugsweise zwischen 0,5 und 15 Gew.-%, bezogen auf das Gesamtgewicht der Zusammensetzung.

13. Zusammensetzung nach einem der vorhergehenden Ansprüche, **dadurch gekennzeichnet, dass** die hydrophoben Siliciumdioxid-Aerogel-Teilchen eine spezifische Oberfläche pro Masseneinheit (S_{M}) im Bereich von 500 bis 1500 m²/g, vorzugsweise von 600 bis 1200 m²/g und noch besser von 600 bis 800 m²/g und eine als volumenmittlerer Durchmesser (D[0,5]) ausgedrückte Größe im Bereich von 1 bis 1500 µm, vorzugsweise von 1 bis 1000 µm, weiter bevorzugt von 1 bis 100 µm, insbesondere 1 bis 30 µm, weiter bevorzugt 5 bis 25 µm, noch besser 5 bis 20 µm und noch besser 5 bis 15 µm aufweisen.

14. Zusammensetzung nach einem der vorhergehenden Ansprüche, **dadurch gekennzeichnet, dass** die hydrophoben Aerogel-Teilchen eine gemäß der in der Beschreibung definierten Methode am Wet Point gemessene Ölabsorptionskapazität im Bereich von 5 bis 18 ml/g, vorzugsweise 6 bis 15 ml/g und noch besser 8 bis 12 ml/g Teilchen aufweisen.

15. Zusammensetzung nach einem der vorhergehenden Ansprüche, **dadurch gekennzeichnet, dass** die hydrophoben Aerogel-Teilchen eine gemäß der in der Beschreibung definierten Methode gemessene Klopfdichte im Bereich von 0,02 g/cm³ bis 0,10 g/cm³ und vorzugsweise von 0,03 g/cm³ bis 0,10 g/cm³ aufweisen.

16. Zusammensetzung nach einem der vorhergehenden Ansprüche, **dadurch gekennzeichnet, dass** es sich bei den hydrophoben Siliciumdioxid-Aerogel-Teilchen um mit Trimethylsilylgruppen oberflächenmodifizierte hydrophobe Siliciumdioxid-Aerogel-Teilchen, vorzugsweise hydrophobe Siliciumdioxid-Aerogel-Teilchen mit dem INCI-Namen Silica silylate, handelt.

17. Zusammensetzung nach einem der vorhergehenden Ansprüche, **dadurch gekennzeichnet, dass** die hydrophoben Aerogel-Teilchen in einem Wirkstoffgehalt im Bereich von 0,1 bis 15 Gew.-%, vorzugsweise von 0,1 bis 10 Gew.-%, vorzugsweise von 0,1 bis 6 Gew.-% und weiter bevorzugt von 0,2 bis 4 Gew. - %, bezogen auf das Gesamtgewicht der Zusammensetzung, vorliegen.

18. Kosmetisches Verfahren zum Schminken und/oder Pflegen der Haut und/oder der Lippen, bei dem man auf die Haut und/oder die Lippen eine Zusammensetzung gemäß einem der vorhergehenden Ansprüche aufbringt.

## Revendications

1. Composition cosmétique liquide, de préférence pour le maquillage et/ou le soin des lèvres et/ou de la peau, comprenant, dans un milieu physiologiquement acceptable, au moins une phase grasse renfermant :
- au moins une huile non volatile,
- au moins un acide gras linéaire hydroxylé en C₁₈-C₂₄, de préférence tel que l'acide 12-hydroxystéarique,
- au moins une cire additionnelle différente dudit acide gras linéaire hydroxylé en C₁₈-C₂₄,
- au moins un copolymère de vinylpyrrolidone et d'au moins une oléfine en C₂-C₄₀ ;
- au moins des particules d'aérogel de silice hydrophobe :
- ladite composition comprenant éventuellement de l'eau à moins de 5% en poids par rapport au poids total de la composition et dé préférence étant anhydre.

2. Composition selon la revendication précédente, **caractérisée en ce qu'**elle comprend une teneur en acide(s) gras linéaire(s) hydroxylé(s) en C₁₈-C₂₄, allant de 0,1 à 5 % en poids, et mieux de préférence de 0,1 à 4 % en poids et de préférence de 0,5 à 3 % en poids et par rapport au poids total de la composition.

3. Composition selon l'une quelconque des revendications précédentes, **caractérisée en ce que** la ou les cires additionnelles sont choisies parmi les cires apolaires ; les cires polaires telles que les cires ester, les cires alcool ou encore les cires siliconées ; ou leurs mélanges, et de préférence parmi les cires correspondant aux esters totaux d'un acide carboxylique en C₁₆-C₃₀, saturé, éventuellement hydroxylé, avec le glycérol, comme la trihydroxystearine ; la cire d'abeille ; la cire d'abeille synthétique ; la cire d'abeille polyglycérolée ; la cire de carnauba ; la cire de candellila ; la cire de lanoline oxypropylénée ; la cire de son de riz ; la cire d'Ouricury ; la cire d'Alfa ; la cire de fibres de liège ; la cire de canne à sucre ; la cire du Japon ; la cire de sumac; la cire de montan ; la cire d'Orange ; la cire de Laurier ; la cire de Jojoba hydrogénée, seules ou en mélange ; parmi les cires de polyéthylène, les cires de polyméthylène, l'ozokerite, les cires microcristallines, seules ou en mélanges.

4. Composition selon la revendication précédente, **caractérisée en ce que** ladite cire additionnelle est choisie parmi les cires présentant un point de fusion d'au moins 60°C.

5. Composition selon l'une quelconque des revendications précédentes, **caractérisée en ce que** la teneur en cire(s) additionnelle(s) est comprise entre 0,1 et 10 % en poids, de préférence entre 0,5% et 7% en poids, encore plus particulièrement entre 0,5 et 5 %en poids, par rapport au poids total de la composition.

6. Composition selon l'une quelconque des revendications précédentes, **caractérisée en ce que** le copolymère de vinylpyrrolidone et d'a-oléfine en C2-C40 est de préférence choisi parmi les copolymères de vinylpyrrolidone et d'héxadécène et de vinylpyrrolidone et d'éicosène,

7. Composition selon l'une quelconque des revendications précédentes, **caractérisée en ce que** la teneur en copolymère de villylpyrrolidone et d'oléfine en C₂-C₄₀ est comprise entre 2 et 8 % en poids par rapport au poids de la composition et de préférence entre 2,5 et 7 % en poids par rapport au poids de la composition.

8. Composition selon l'une quelconque des revendications précédentes, **caractérisée en ce que** l'huile non volatile est choisie parmi les huiles hydrocarbonées apolaires, de préférence choisies parmi l'huile de paraffine ou ses dérivés, le squalane, l'isocicosane, l'huile de naphtalène, les polybutylènes, les polyisobutènes hydrogénés ou non, les copolymères décène/butène, les copolymères polybutène/polyisobutène, les polydécènes hydrogénés ou non, leurs mélanges et plus particulièrement parmi les polybutènes, les polyisobutènes, les polyisobutènes hydrogénés, les polydécènes et/ou les polydécènes hydrogénés, et leurs mélanges.

9. Composition selon l'une quelconque des revendications précédentes, **caractérisée en ce qu'**elle comprend une teneur en huile(s) non volatile(s) apolaire comprise entre 5 % et 60 % en poids, en particulier entre 10 % et 45 % en poids, par rapport au poids total de la composition.

10. Composition selon l'une quelconque des revendications précédentes, **caractérisée en ce que** l'huile non volatile est choisie parmi les huiles hydrocarbonées, siliconées ou fluorées polaires et de préférence parmi les huiles hydrocarbonées végétales ou d'origine végétale, les huiles esters, les alcools gras ayant de 12 à 26 atomes de carbone, les acides gras ayant de 12 à 26 atomes de carbone et les huiles siliconées, de préférence choisies parmi les huiles siliconées non phénylées de nom INCI « dimethicone », et leurs mélanges.

11. Composition selon l'une quelconque des revendications précédentes, **caractérisée en ce qu'**elle comprend une teneur en huile(s) non volatile(s) polaire(s) comprise entre 15 % et 90 % en poids, en particulier entre 25 % et 80 % en poids, et de préférence entre 35 et 70 % en poids, par rapport au poids total de la composition.

12. Composition selon l'une quelconque des revendications précédentes, **caractérisée en ce qu'**elle comprend au moins une matière colorante choisie parmi les pigments et/ou les nacres, de préférence en une teneur totale comprise entre 0,1 et 20%, de préférence entre 0,5 et 15% en poids, par rapport au poids total de la composition.

13. Composition selon l'une quelconque des revendications précédentes, **caractérisée en ce que** les particules d'aérogel de silice hydrophobes présentent une surface spécifique par unité de masse (S_{M}) allant de 500 à 1500 m²/g, de préférence de 600 à 1200 m²/g, et mieux de 600 à 800 m²/g, et une taille exprimée en diamètre moyen en volume (D[0,5]) allant de 1 à 1500 µm, de préférence de 1 à 1000 µm, encore plus préférentiellement de 1 à 100 µm, en particulier de 1 à 30 µm, de préférence encore de 5 à 25 µm, mieux de 5 à 20 µm et encore mieux de mieux de 5 à 15 µm.

14. Composition selon l'une quelconque des revendications précédentes, **caractérisée en ce que** les particules d'aérogel hydrophobes présentent une capacité d'absorption d'huile mesurée au WET POINT selon le procédé défini dans la description, allant de 5 à 18 ml/g de particules, de préférence de 6 à 15 ml/g et mieux de 8 à 12 ml/g ;

15. Composition selon l'une quelconque des revendications précédentes, **caractérisée en ce que** les particules d'aérogel hydrophobes présentent une densité tassée mesurée selon le procédé défini dans la description allant de 0,02g/cm³ à 0,10 g/cm³ et de préférence de 0,03g/cm³ à 0,08g/cm³.

16. Composition selon l'une quelconque des revendications précédentes, **caractérisée en ce que** les particules d'aérogel de silice hydrophobes sont des particules d'aérogel de silice hydrophobes modifiées en surface par groupements triméthylsilyle, de préférence des particules d'aérogel de silice hydrophobes de nom INCI Silica silylate.

17. Composition selon l'une quelconque des revendications précédentes, **caractérisée en ce que** les particules d'aérogel hydrophobes sont présentes en une teneur en matière active allant de 0,1 à 15 % en poids, de préférence de 0,1 à 10 % en poids, de préférence de 0,1 à 6 % en poids, de préférence encore de 0,2% à 4 % en poids par rapport au poids total de la composition.

18. Procédé cosmétique de maquillage et/ou de soin de la peau et/ou des lèvres comprenant l'application sur la peau et/ou les lèvres, d'une composition telle que définie dans l'une quelconque des revendications précédentes.
